# EUROPEAN PATENT SPECIFICATION

(11) **EP 1 960 528 B1**
(45) Date of publication and mention of the grant of the patent: **24.04.2013**
(21) Application number: 06835694.8
(22) Date of filing: 12.12.2006
(51) Int. Cl.: C12N 15/82, C12N 9/88, A01H 5/00

(54) **CONSTITUTIVE PLANT PROMOTERS**
KONSTITUTIVE PFLANZENPROMOTOREN
PROMOTEURS DE PLANTE CONSTITUTIFS

(30) Priority: 16.12.2005 WO PCT/NL2005/050083
(43) Date of publication of application: 27.08.2008
(73) Proprietor: KEYGENE N.V., 6708 PW Wageningen (NL)
(72) Inventor: DE BOTH, Michiel, Theodoor, Jan, NL-6708 BG Wageningen (NL); QUAEDVLIEG, Nicolette, Elisabeth, Maria, NL-2251 RR Voorschoten (NL); FIERENS-ONSTENK, Bernarda, Gerharda, Johanna, NL-3905 VS Veenendaal (NL); WACHOWSKI, Ludvik, Kevin, NL-6708 BV Wageningen (NL)
(74) Representative: de Lang, Robbert-Jan
(86) International application number: PCT/NL2006/050314
(87) International publication number: WO 2007/069894

(56) References cited:
- EP-A2- 0 342 926
- WO-A-91/09948
- WO-A2-99/66057
- GUTIERREZ-ALCALA GLORIA ET AL: "A versatile promoter for the expression of proteins in glandular and non-glandular trichomes from a variety of plants" JOURNAL OF EXPERIMENTAL BOTANY, vol. 56, no. 419, September 2005 (2005-09), pages 2487-2494, XP002406257 ISSN: 0022-0957
- DATABASE EMBL [Online] 12 May 2004 (2004-05-12), "Lycopersicon esculentum clone 133047R, mRNA sequence." XP002406259 retrieved from EBI accession no. EM_PRO:BT014000 Database accession no. BT014000
- ODELL J T ET AL: "IDENTIFICATION OF DNA SEQUENCES REQUIRED FOR ACTIVITY OF THE CAULIFLOWER MOSAIC VIRUS 35S PROMOTER" NATURE, NATURE PUBLISHING GROUP, LONDON, GB, vol. 313, 28 February 1985 (1985-02-28), pages 810-812, XP002915192 ISSN: 0028-0836 cited in the application
- JOST R ET AL: "Genomic and functional characterization of the oas gene family encoding O-acetylserine (thiol) lyases, enzymes catalyzing the final step in cysteine biosynthesis in Arabidopsis thaliana" GENE, ELSEVIER, AMSTERDAM, NL, vol. 253, no. 2, 8 August 2000 (2000-08-08), pages 237-247, XP004215728 ISSN: 0378-1119

## Description

### FIELD OF THE INVENTION

The present invention relates to a class of strong, constitutive plant promoters and their uses. The promoters may be used for expression of homologous or heterologous proteins in plants or plant cells, or for the expression of active nucleic acid molecules, such as sense and/or anti-sense RNA. Provided are nucleic acid sequences having promoter activity, as well as chimeric genes, vectors and recombinant (transgenic) cells and organism comprising these. Also provided are methods for making transgenic cells and organisms, especially plants and plant cells, comprising the promoter.

### BACKGROUND OF THE INVENTION

A large number of plant promoters are known in the art, which are useful tools for expressing proteins or peptides in transgenic plants or plant cells or for silencing genes or gene families. These include constitutive promoters, inducible promoters, developmentally regulated promoters, tissue preferred or tissue specific promoters. Examples of commonly used constitutive promoters are the following: the 35S promoters or enhanced 35S promoters (the "35S promoters") of the cauliflower mosaic virus (CaMV) of isolates CM 1841 (Gardner et al., 1981, Nucleic Acids Research 9, 2871-2887), CabbB-S (Franck et al., 1980, Cell 21, 285-294) and CabbB-JI (Hull and Howell, 1987, Virology 86, 482-493); the 35S promoter described by Odell et al. (1985, Nature 313, 810-812) or in US5164316, promoters from the ubiquitin family (e.g. the maize ubiquitin promoter of Christensen et al., 1992, Plant Mol. Biol. 18, 675-689, EP 0 342 926, see also Cornejo et al. 1993, Plant Mol. Biol. 23, 567-581), the gos2 promoter (de Pater et al., 1992 Plant J. 2, 837-844), the emu promoter (Last et al., 1990, Theor. Appl. Genet. 81,581-588), *Arabidopsis* actin promoters such as the promoters described by An et al. (1996, Plant J. 10, 107-121), rice actin promoters such as the promoters described by Zhang et al.(1991, The Plant Cell 3, 1155-1165) and the promoter described in US 5,641,876 or the rice actin 2 promoter as described in WO 00/70067; promoters of the Cassava vein mosaic virus (WO 97/48819, Verdaguer et al. 1998, Plant Mol. Biol. 37, 1055-1067), the pPLEX series of promoters from Subterranean Clover Stunt Virus (WO 96/06932, particularly the S7 promoter), an alcohol dehydrogenase promoter, e.g., pAdh1S (GenBank accession numbers X04049, X00581), the Figwort Mosaic Virus promoter described in US 6 051 753 and in EP 426 641, histone gene promoters, such as the Ph4a748 promoter from *Arabidopsis* (Plant Mol. Biol. 8: 179-191), the CoYMV (Commelina Yellow Mottle Virus) promoter (Medberry et al. 1992, The Plant Cell 4:185-192), or others.

Gutiérrez-Alcalá et al. (2005. J. Exp. Bot. Vol. 56:2487-2494) disclose transgenic plants comprising an OASA1 promoter able to drive expression in glandular trichomes.

GenBank Accession no. BT014000 discloses an mRNA sequence from Lycopersicon esculentum clone 133047R.

EP 0 342 926 discloses an ubiquitin promoter to control expression of a DNA coding sequence.

However, one common drawback of known constitutive promoters is that they frequently still show variation in their activity, such as organ- or developmentally regulated expression or stress induced alterations in expression. The present inventors found that the activity of the CaMV 35S promoter in transgenic plants was sensitive to abiotic stress, especially heat stress caused when the transgenic plants were grown in the field in Spain. There is, therefore, a need to provide new constitutive promoters, which are insensitive to one or more biotic and/or abiotic stresses. In addition, it is desirable from a regulatory point of view to use promoters derived from plants in the generation of transgenic plants.

In addition, viral promoters derived from viruses capable of infecting plants are less preferred for the transformation of host plant species, as infection of the plants with the virus may cause silencing of the transgenic promoter (Seemanpillai et al., 2003, Mol Plant Microbe Interact. 16(5): 429-38; Al-Kaff et al., 2000, Nat Biotechnol. 18: 995-9). Different constitutive promoters are also needed for gene stacking approaches, as the use of several identical promoters may result in silencing (Yang et al., 2005, Plant Mol Biol. 58: 351-66).

### SUMMARY OF THE INVENTION

Provided is a transgenic plant or plant cell or plant tissue or organ comprising a chimeric gene integrated in its genome, characterized in that said chimeric gene comprises a constitutive promoter operably linked to a homologous or heterologous nucleic acid sequence, wherein a homologous nucleic acid sequence is found naturally in said plant or plant cell or plant tissue or organ, and wherein a heterologous nucleic acid sequence is not naturally found in said plant or plant cell or plant tissue or organ, wherein the constitutive promoter is selected from the group of: the nucleic acid sequence of SEQ ID NO: 1 or SEQ ID NO: 2;
(a) a functional fragment of SEQ ID NO: 1 or SEQ ID NO: 2, said functional fragment being capable of conferring constitutive transcription in plant cells, organs and plants;
(b) a nucleic acid sequence comprising at least 70% sequence identity with SEQ ID NO: 1 or 2 over the full length, said nucleic acid sequence being capable of conferring constitutive transcription in plant cells, organs and plants;
(c) a functional fragment of the nucleic acid sequence of (c),
wherein said promoter is not the promoter of the OASA1 gene of Arabidopsis thaliana, or a fragment thereof.

Also provided is an isolated nucleic acid sequence having constitutive promoter activity when introduced into plant cells, wherein said nucleic acid sequence comprising a sequence selected from:
(a) the nucleic acid sequence of SEQ ID NO: 1 or SEQ ID NO: 2,
(b) a fragment of SEQ ID NO: 1 or SEQ ID NO: 2, said fragment being capable of conferring constitutive transcription in plant cells, organs and plants;
(c) a nucleic acid sequence comprising at least 70% sequence identity with SEQ ID NO: 1 or 2 over the full length, said nucleic acid sequence being capable of conferring constitutive transcription in plant cells, organs and plants;
(d) a fragment of the nucleic acid sequence of (c), said fragment being capable of conferring constitutive transcription in plant cells, organs and plants.

Vectors, chimeric genes and host cells comprising the above sequences are also an embodiment of the invention.

Described is embodiment the use of the promoter of a cytosolic plant cysteine synthase gene for the constitutive expression of a sense and/or antisense nucleic acid sequence in a transgenic plant or plant cell or plant tissue or organ.

Further, a method is described for making a transgenic plant or plant cell, comprising the steps of:
(a) generating a chimeric gene comprising a promoter as described above, operably linked to a nucleic acid sequence to be transcribed, and optionally further linked to a 3'UTR nucleic acid sequence, or a vector comprising such a chimeric gene;
(b) transforming a plant or plant cell with said chimeric gene or vector; and, optionally,
(c) regenerating transgenic plants or plant cells.

In yet another embodiment an isolated nucleic acid molecule comprising the nucleotide sequence of SEQ ID NO: 3 is provided.

### GENERAL DEFINITIONS

The term "nucleic acid sequence" (or nucleic acid molecule) refers to a DNA or RNA molecule in single or double stranded form, particularly a DNA having promoter activity according to the invention or a DNA encoding a protein or protein fragment. An "isolated nucleic acid sequence" refers to a nucleic acid sequence which is no longer in the natural environment from which it was isolated, e.g. the nucleic acid sequence in a bacterial host cell or in the plant nuclear or plastid genome.

The terms "protein" or "polypeptide" are used interchangeably and refer to molecules consisting of a chain of amino acids, without reference to a specific mode of action, size, 3-dimensional structure or origin. A "fragment" or "portion" of a protein may thus still be referred to as a "protein". An "isolated protein" is used to refer to a protein which is no longer in its natural environment, for example *in vitro* or in a recombinant bacterial or plant host cell.

The term "gene" means a DNA sequence comprising a region (transcribed region), which is transcribed into an RNA molecule (e.g. an mRNA) in a cell, operably linked to suitable transcription regulatory regions (e.g. a promoter). A gene may thus comprise several operably linked sequences, such as a promoter, a 5' non-translated leader sequence (also referred to as 5'UTR, which corresponds to the transcribed mRNA sequence upstream of the translation start codon) comprising e.g. sequences involved in translation initiation, a (protein) coding region (cDNA or genomic DNA) and a 3'non-translated sequence (also referred to as 3' untranslated region, or 3'UTR) comprising e.g. transcription termination sites and polyadenylation site (such as e.g. AAUAAA or variants thereof).

A "chimeric gene" (or recombinant gene) refers to any gene, which is not normally found in nature in a species, in particular a gene in which one or more parts of the nucleic acid sequence are present that are not associated with each other in nature. For example the promoter is not associated in nature with part or all of the transcribed region or with another regulatory region. The term "chimeric gene" is understood to include expression constructs in which a promoter or transcription regulatory sequence is operably linked to one or more sense sequences (e.g. coding sequences) or to an antisense (reverse complement of the sense strand) or inverted repeat sequence (sense and antisense, whereby the RNA transcript forms double stranded RNA upon transcription).

A "3' UTR" or "3' non-translated sequence" (also often referred to as 3' untranslated region, or 3'end) refers to the nucleic acid sequence found downstream of the coding sequence of a gene, which comprises, for example, a transcription termination site and (in most, but not all eukaryotic mRNAs) a polyadenylation signal (such as e.g. AAUAAA or variants thereof). After termination of transcription, the mRNA transcript may be cleaved downstream of the polyadenylation signal and a poly(A) tail may be added, which is involved in the transport of the mRNA to the cytoplasm (where translation takes place).

"Expression of a gene" refers to the process wherein a DNA region, which is operably linked to appropriate regulatory regions, particularly a promoter, is transcribed into an RNA, which is biologically active, i.e. which is capable of being translated into a biologically active protein or peptide (or active peptide fragment) or which is active itself (e.g. in posttranscriptional gene silencing or RNAi). An active protein in certain embodiments refers to a protein having a dominant-negative function due to a repressor domain being present. The coding sequence is preferably in sense-orientation and encodes a desired, biologically active protein or peptide, or an active peptide fragment. In gene silencing approaches, the DNA sequence is preferably present in the form of an antisense DNA or an inverted repeat DNA, comprising a short sequence of the target gene in antisense or in sense and antisense orientation. "Ectopic expression" refers to expression in a tissue in which the gene is normally not expressed.

A "transcription regulatory sequence" is herein defined as a nucleic acid sequence that is capable of regulating the rate of transcription of a nucleic acid sequence operably linked to the transcription regulatory sequence. A transcription regulatory sequence as herein defined will thus comprise all of the sequence elements necessary for initiation of transcription (promoter elements), for maintaining and for regulating transcription, including e.g. attenuators or enhancers, but also silencers. Although mostly the upstream (5') transcription regulatory sequences of a coding sequence are referred to, regulatory sequences found downstream (3') of a coding sequence are also encompassed by this definition.

As used herein, the term "promoter" refers to a nucleic acid fragment that functions to control the transcription of one or more genes, located upstream (5') with respect to the direction of transcription of the transcription initiation site of the gene (the transcription start is referred to as position +1 of the sequence and any upstream nucleotides relative thereto are referred to using negative numbers), and is structurally identified by the presence of a binding site for DNA-dependent RNA polymerase, transcription initiation sites and any other DNA domains (cis acting sequences), including, but not limited to transcription factor binding sites, repressor and activator protein binding sites, and any other sequences of nucleotides known to one of skill in the art to act directly or indirectly to regulate the amount of transcription from the promoter. Examples of eukaryotic cis acting sequences upstream of the transcription start (+1) include the TATA box (commonly at approximately position -20 to -30 of the transcription start), the CAAT box (commonly at approximately position -75 relative to the transcription start), 5'enhancer or silencer elements, etc. A "constitutive" promoter is a promoter that is active in most tissues (or organs) under most physiological and developmental conditions. More preferably, a constitutive promoter is active under essentially all physiological and developmental conditions in all major organs, such as at least the leaves, stems, roots, seeds, fruits and flowers. Most preferably, the promoter is active in all organs under most (preferably all) physiological and developmental conditions.

An "inducible" promoter is a promoter that is physiologically (e.g. by external application of certain compounds) or developmentally regulated. A "tissue specific" promoter is only active in specific types of tissues or cells. The promoter activity can therefore be described by referring to the circumstances under which the promoter confers transcription of the nucleic acid sequence operably linked downstream (3') of the promoter. A "promoter which has constitutive activity" or which is "constitutive" in a plant or plant cell refers, therefore, to a nucleic acid sequence which confers transcription in the plant or plant cells in most tissues (or organs) under most physiological and developmental conditions. A promoter which is "insensitive to one or more biotic and/or abiotic stresses" or whose activity "is not reduced when exposed to one or more biotic and/or abiotic stress conditions" refers to a nucleic acid sequence having promoter activity under normal physiological and developmental conditions, and whereby the activity is not, or at least not significantly, reduced quantitatively when biotic and/or abiotic stress is exerted on the organism (e.g. plant) or cells or tissues or organs comprising the promoter.

"Stress" refers to conditions or pressures of physical, chemical or biological origin acting on a plant or plant cells which may result in yield loss and/or quality loss of a plant, but which is not lethal to the plant.

"Non-stress conditions" refer herein to conditions under which physiology and development are normal or optimal.

"Biotic stress" refers to stress caused by biotic (live) agents, such as fungi, viruses, mycoplasma like organisms, insects, bacteria, nematodes etc. (i.e. especially plant pests and pathogens).

"Abiotic stress" refers to stress caused by abiotic (non-living) agents, such as temperature stress (cold/freezing, heat), salinity (salt), wind, metals, day-length (photoperiod), water-stress (such as too little or too much water availability, i.e. drought, dehydration, water-logging, etc.), wounding, radiation, etc.

As used herein, the term "operably linked" refers to a linkage of polynucleotide elements in a functional relationship. A nucleic acid is "operably linked" when it is placed into a functional relationship with another nucleic acid sequence. For instance, a promoter, or a transcription regulatory sequence, is operably linked to a coding sequence if it affects the transcription of the coding sequence. Operably linked means that the DNA sequences being linked are typically contiguous and, where necessary to join two protein encoding regions, contiguous and in reading frame so as to produce a "chimeric protein". A "chimeric protein" or "hybrid protein" is a protein composed of various protein "domains" (or motifs) which is not found as such in nature but which are joined to form a functional protein, which displays the functionality of the joined domains (for example a DNA binding domain or a repression of function domain leading to a dominant negative function). A chimeric protein may also be a fusion protein of two or more proteins occurring in nature. The term "domain" as used herein means any part(s) or domain(s) of the protein with a specific structure or function that can be transferred to another protein for providing a new hybrid protein with at least the functional characteristic of the domain.

The term "target peptide" refers to amino acid sequences which target a protein to intracellular organelles such as plastids, preferably chloroplasts, mitochondria, or to the extracellular space (secretion signal peptide). A nucleic acid sequence encoding a target peptide may be fused (in frame) to the nucleic acid sequence encoding the amino terminal end (N-terminal end) of the protein.

A "nucleic acid construct" or "vector" is herein understood to mean a man-made nucleic acid molecule resulting from the use of recombinant DNA technology and which is used to deliver exogenous DNA into a host cell. The vector backbone may for example be a binary or superbinary vector (see e.g. US 5,591,616, US2002138879 and WO 95/06722), a co-integrate vector or a T-DNA vector, as known in the art and as described elsewhere herein, into which a chimeric gene is integrated or, if a suitable transcription regulatory sequence / promoter is already present, only a desired nucleic acid sequence (e.g. a coding sequence, an antisense or an inverted repeat sequence) is integrated downstream of the transcription regulatory sequence / promoter. Vectors usually comprise further genetic elements to facilitate their use in molecular cloning, such as e.g. selectable markers, multiple cloning sites and the like (see below).

A "host cell" or a "recombinant host cell" or "transformed cell" are terms referring to a new individual cell (or organism), arising as a result of the introduction into said cell of at least one nucleic acid molecule, especially comprising a chimeric gene encoding a desired protein or a nucleic acid sequence which upon transcription yields an antisense RNA or an inverted repeat RNA (or hairpin RNA) for silencing of a target gene/gene family. The host cell is preferably a plant cell, but may also be a bacterial cell, a fungal cell (including a yeast cell), etc. The host cell may contain the nucleic acid construct as an extra-chromosomally (episomal) replicating molecule, or more preferably, comprises the chimeric gene integrated in the nuclear or plastid genome of the host cell. The term "selectable marker" is a term familiar to one of ordinary skill in the art and is used herein to describe any genetic entity which, when expressed, can be used to select for a cell or cells containing the selectable marker. Selectable marker gene products confer, for example, antibiotic resistance, or more preferably, herbicide resistance or another selectable trait such as a phenotypic trait (e.g. a change in pigmentation) or a nutritional requirement. The term "reporter" is mainly used to refer to visible markers, such as green fluorescent protein (GFP), eGFP, luciferase, GUS and the like.

The term "ortholog" of a gene or protein refers herein to the homologous gene or protein found in another species, which has the same function as the gene or protein, but (usually) diverged in sequence from the time point on when the species harbouring the genes diverged (i.e. the genes evolved from a common ancestor by speciation). Orthologs of a gene from one plant species may thus be identified in other plant species based on both sequence comparisons (e.g. based on percentages sequence identity over the entire sequence or over specific domains) and functional analysis.

The terms "homologous" and "heterologous" refer to the relationship between a nucleic acid or amino acid sequence and its host cell or organism, especially in the context of transgenic organisms. A homologous sequence is thus naturally found in the host species (e.g. a tomato plant transformed with a tomato gene), while a heterologous sequence is not naturally found in the host cell (e.g. a tomato plant transformed with a sequence from potato plants). Depending on the context, the term "homolog" or "homologous" may alternatively refer to sequences which are descendent from a common ancestral sequence (e.g. they may be orthologs).

"Stringent hybridisation conditions" can be used to identify nucleotide sequences, which are substantially identical to a given nucleotide sequence. The stringency of the hybridization conditions are sequence dependent and will be different in different circumstances. Generally, stringent conditions are selected to be about 5°C lower than the thermal melting point (Tₘ) for the specific sequences at a defined ionic strength and pH. The Tₘ is the temperature (under defined ionic strength and pH) at which 50% of the target sequence hybridises to a perfectly matched probe. Typically stringent conditions will be chosen in which the salt concentration is about 0.02 molar at pH 7 and the temperature is at least 60°C. Lowering the salt concentration and/or increasing the temperature increases stringency. Stringent conditions for RNA-DNA hybridisations (Northern blots using a probe of e.g. 100 nt) are for example those which include at least one wash in 0.2X SSC at 63°C for 20 min, or equivalent conditions. Stringent conditions for DNA-DNA hybridisation (Southern blots using a probe of e.g. 100nt) are for example those which include at least one wash (usually 2) in 0.2X SSC at a temperature of at least 50°C, usually about 55°C, for 20 min, or equivalent conditions. See also Sambrook *et al.* (1989) and Sambrook and Russell (2001). "Sequence identity" and "sequence similarity" can be determined by alignment of two peptide or two nucleotide sequences using global or local alignment algorithms, depending on the length of the two sequences. Sequences of similar lengths are preferably aligned using a global alignment algorithms (e.g. Needleman Wunsch) which aligns the sequences optimally over the entire length, while sequences of substantially different lengths are preferably aligned using a local alignment algorithm (e.g. Smith Waterman). Sequences may then be referred to as "substantially identical" or "essentially similar" when they (when optimally aligned by for example the programs GAP or BESTFIT using default parameters) share at least a certain minimal percentage of sequence identity (as defined below). GAP uses the Needleman and Wunsch global alignment algorithm to align two sequences over their entire length (full length), maximizing the number of matches and minimizing the number of gaps. A global alignment is suitably used to determine sequence identity when the two sequences have similar lengths. Generally, the GAP default parameters are used, with a gap creation penalty = 50 (nucleotides) / 8 (proteins) and gap extension penalty = 3 (nucleotides) / 2 (proteins). For nucleotides the default scoring matrix used is nwsgapdna and for proteins the default scoring matrix is Blosum62 (Henikoff & Henikoff, 1992, PNAS 89, 915-919). Sequence alignments and scores for percentage sequence identity may be determined using computer programs, such as the GCG Wisconsin Package, Version 10.3, available from Accelrys Inc., 9685 Scranton Road, San Diego, CA 92121-3752 USA, or using open source software, such as the program "needle" (using the global Needleman Wunsch algorithm) or "water" (using the local Smith Waterman algorithm) in EmbossWIN version 2.10.0, using the same parameters as for GAP above, or using the default settings (both for 'needle' and for 'water' and both for protein and for DNA alignments, the default Gap opening penalty is 10.0 and the default gap extension penalty is 0.5; default scoring matrices are Blossum62 for proteins and DNAFull for DNA). When sequences have a substantially different overall lengths, local alignments, such as using the Smith Waterman algorithm, are preferred. Alternatively percentage similarity or identity may be determined by searching against public databases, using algorithms such as FASTA, BLAST, etc.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one". It is further understood that, when referring to "sequences" herein, generally the actual physical molecules with a certain sequence of subunits (e.g. amino acids) are referred to.

Whenever reference to a "plant" or "plants" (or a plurality of plants) according to the invention is made, it is understood that also plant parts (cells, tissues or organs, seeds, severed or harvested parts, leaves, seedlings, flowers, pollen, fruit, stems, roots, callus, protoplasts, etc), progeny or clonal propagations of the plants which retain the distinguishing characteristics of the parents (e.g. presence of a trans-gene), such as seed obtained by selfing or crossing, e.g. hybrid seed (obtained by crossing two inbred parental lines), hybrid plants and plant parts derived therefrom are encompassed herein, unless otherwise indicated.

### DETAILED DESCRIPTION

When testing transgenic plants comprising the CaMV 35S promoter (single 35S promoter, described by Franck et al., 1980, Cell 21, 285-294) in field trials in Spain it was found that the activity of the 35S promoter was influenced by high summer temperatures. In older leaves, a total loss of expression occurred. The present inventors, therefore, initiated a program for isolating plant promoters which have constitutive activity in plant cells and organs, and which are essentially insensitive to one or more biotic and/or abiotic stresses, as such a promoter is desired for controlled expression of nucleic acid sequences in transgenic plants, especially in plants which may be exposed to one or more stress conditions during their development or at maturity. Using cDNA-AFLP, genes of strong and constitutive promoters were isolated from tomato plants, which retained strong, constitutive transcription profiles under stress conditions, such as drought stress, cold stress, heat stress, pathogen stress (CMV infection), day-length variation, radiation (e.g. UV induced stress), water-stress (over watering and under watering), etc. (see Examples). By isolating the corresponding promoters, a class of promoters was found which conferred (or is capable of conferring) strong, constitutive expression in plants. The promoter strength was at least equivalent, and in some host species (tomato) significantly higher than that of the CaMV 35S promoter. Further, the promoters do not show reduced activity (i.e. remain strong and constitutive) when plants or plant parts comprising these are subjected to various biotic and/or abiotic stress conditions. These promoters are herein referred to as the "AA6 promoters".

### Nucleic acid sequences, chimeric genes and vectors

In one embodiment isolated nucleic acid sequences (preferably genomic or synthetic DNA sequences), having promoter activity in plant cells, are provided which show strong, constitutive transcriptional activity in plants and plant tissues or organs. The promoters are active in preferably all plant organs, but at least in the major organs and the promoter strength is at least essentially equal to that of 35S (or stronger) (the CaMV 35S promoter described by Franck *et al.,* supra). Preferably the promoter activity of the nucleic acid sequences is not reduced, or at least not significantly reduced, when the transgenic plant, or plant tissue or organ, is subjected to one or more abiotic and/or biotic stresses. A significant reduction in this respect refers to a statistically significant (quantitative) reduction of promoter activity by 1% or more (e.g. 2%, 3%, 5%, 10%, etc., up to 100%) compared to the activity in the same tissues or organs under non-stress conditions. Thus, preferably the promoters remain strong and constitutive (preferably in all organs, especially at least the major plant organs) under stress conditions.

In one embodiment a constitutive AA6 promoter is provided comprising SEQ ID NO: 1 ("3kb" promoter) or SEQ ID NO: 2 ("5kb" promoter). Described are SEQ ID NO: 6-9 (SEQ ID NO: 6 and 8 are "3kb" promoters and SEQ ID NO: 7 and 9 are "5kb"promoters with some nucleotide ambiguities or slightly different sequences compared to SEQ ID NO: 1 and 2), or the promoter sequences cloned into pKG8135 (CBS120175) or pKG8137 (CBS120176), or active (functional) fragments of any of these which have promoter activity at least in plants, such as fragments of at least 200, 300, 400, 500, 600, 800, 900, 1000, 1200, 1500, 2000, 2500, 2800, 2900, 3000, 3500, 4000, 4500 or more consecutive nucleotides of SEQ ID NO: 1 or 2, SEQ ID NO: 6-9, or promoters within pKG8135 (CBS120175) or pKG8137 (CBS120176). "Active fragments" or "functional fragments", or "fragments having promoter activity" refer to nucleic acid fragments which are capable of conferring constitutive transcription in plant cells, organs and plants, preferably in at least the same tissues and organs as SEQ ID NO: 1 and 2, SEQ ID NO: 6-9, or promoters within pKG8135 (CBS120175) or pKG8137 (CBS 120176). This can be tested as described below, by transforming a cell with such a fragment, preferably operably linked to a reporter gene, and assaying the promoter activity qualitatively (spatio-temporal transcription) and/or quantitatively.

When referring herein to SEQ ID NO: 1 it is understood that reference is also made to any of SEQ ID NO: 6 or 8, or the sequence present in pKG8135 (CBS120175) and that the sentence can be read to refer to any of these promoter sequences. Likewise, when referring herein to SEQ ID NO: 2 it is understood that reference is also made to any of SEQ ID NO: 7 or 9, or the sequence present in pKG8137 (CBS120175) and that the sentence can be read to refer to any of these promoter sequences.

In one embodiment, the strength of the promoter fragments is quantitatively essentially identical to, or higher than, that of SEQ ID NO: I and/or 2 (and therefore also that of 35S). As SEQ ID NO: 1 and 2 are sequences directly upstream of the translation initiation codon (AUG on the mRNA or ATG on the DNA), active fragments are preferably generated by deletions at the 5' end of SEQ ID NO: 1 and/or 2. The fragments, therefore, preferably comprise at least 200, 300, 400, 500, etc. nucleotides (as above) of the 3' region of SEQ ID NO: 1 or 2. Obviously, DNA fragments may be generated in a number of ways, e.g. using *de novo* DNA synthesis, or restriction enzymes, or terminal nucleases, etc.

However, removal of certain *cis*-acting elements (such as enhancer sequences) may result in lower promoter activity. Therefore, in a different embodiment the strength of the promoter fragments is quantitatively lower than that of SEQ ID NO: 1 and/or 2 (and therefore also that of 35S). For some applications, promoters with reduced strength are preferred. A skilled person can easily determine the activity of the full length promoters and any promoter fragments using methods known in the art, and can compare the strength and tissue specificity to that of 35S or to that of the promoters provided herein. For example Medberry et al. (Plant Cell, 1992, Vol. 4: 185-192) describe methods for comparing promoter strength using transient expression assays.

The promoters of SEQ ID NO: 1 and 2, and functional fragments thereof, are preferably insensitive to at least one (but preferably more, most preferably any) biotic and/or abiotic stress to which the plant or plant cell(s), tissues or organs comprising the promoter may be exposed (see below). Thus, activity remains constitutive and strong during exposure to stress conditions.

Also provided are "variants" of the above AA6 promoters and functional fragments of such variants. These variants include nucleic acid sequences essentially similar to SEQ ID NO: 1 and/or SEQ ID NO: 2 (and functional fragments of these variant sequences, as described above), and which have constitutive promoter activity, i.e. which are also capable of providing constitutive transcription in plants, plant cells, tissues or organs, most preferably in at least the same tissues and organs as SEQ ID NO: 1 and 2. Sequences which are "essentially similar" to SEQ ID NO: 1 and/or 2 are nucleic acid sequences comprising at least about 70, 75, 80, 85, 90, 95, 96, 97, 98, 99% or more nucleic acid sequence identity to SEQ ID NO: 1 and/or to SEQ ID NO: 2 over the full length, using the Needleman and Wunsch Pairwise alignment (program "GAP" in GCG or "needle" in Embosswin, version 2.10.0) with gap creation penalty = 50 and gap extension penalty = 3) and which have promoter activity in plants or plant cells. In a preferred embodiment, the activity of the variants (and functional fragments) is strong in all of the tissues and organs, i.e. quantitatively at least as strong (or stronger) than SEQ ID NO: 1 or 2. In a further embodiment the activity of these variants (and functional fragments thereof) is insensitive to one or more biotic and/or abiotic stresses, i.e. remains strong and constitutive.

It is clear that many methods can be used to identify, synthesise or isolate variants or functional fragments of the nucleic acid sequences provided herein, such as nucleic acid hybridization, PCR technology, *in silico* analysis and nucleic acid synthesis, and the like. For example, nucleic acid hybridization can be used to identify DNA sequences in other plant species or varieties which hybridize to SEQ ID NO: 1 or 2, or to fragments of these, under stringent or moderately stringent hybridization conditions. For example Medberry et al. (Plant Cell, 1992, Vol. 4: 185-192) describe methods for comparing promoter strength using transient expression assays.

The promoters of SEQ ID NO: I and 2, and functional fragments thereof, are preferably insensitive to at least one (but preferably more, most preferably any) biotic and/or abiotic stress to which the plant or plant cell(s), tissues or organs comprising the promoter may be exposed (see below). Thus, activity remains constitutive and strong during exposure to stress conditions.

Also provided are "variants" of the above AA6 promoters and functional fragments of such variants. These variants include nucleic acid sequences essentially similar to SEQ ID NO: 1 and/or SEQ ID NO: 2 (and functional fragments of these variant sequences, as described above), and which have constitutive promoter activity, i.e. which are also capable of providing constitutive transcription in plants, plant cells, tissues or organs, most preferably in at least the same tissues and organs as SEQ ID NO: 1 and 2. Sequences which are "essentially similar" to SEQ ID NO: 1 and/or 2 are nucleic acid sequences comprising at least about 50, 55, 60, 65, 70, 75, 80, 85, 90, 95, 96, 97, 98, 99% or more nucleic acid sequence identity to SEQ ID NO: I and/or to SEQ ID NO: 2 (over the full length), using the Needleman and Wunsch Pairwise alignment (program "GAP" in GCG or "needle" in Embosswin, version 2.10.0) with gap creation penalty = 50 and gap extension penalty = 3) and which have promoter activity in plants or plant cells. In a preferred embodiment, the activity of the variants (and functional fragments) is strong in all of the tissues and organs, i.e. quantitatively at least as strong (or stronger) than SEQ ID NO: 1 or 2. In a further embodiment the activity of these variants (and functional fragments thereof) is insensitive to one or more biotic and/or abiotic stresses, i.e. remains strong and constitutive.

It is clear that many methods can be used to identify, synthesise or isolate variants or functional fragments of the nucleic acid sequences provided herein, such as nucleic acid hybridization, PCR technology, *in silico* analysis and nucleic acid synthesis, and the like. For example, nucleic acid hybridization can be used to identify DNA sequences in other plant species or varieties which hybridize to SEQ ID NO: I or 2, or to fragments of these, under stringent or moderately stringent hybridization conditions.

Alternatively, sequence databases can be screened *in silico* for variant sequences using known algorithms, such as BLAST, FASTA, etc. In this way it is feasible to isolate variant sequences from other plant species or other varieties of tomato, or from other organisms altogether. Especially included herein are the promoters of other alleles of the same gene (cytosolic cysteine synthase gene and orthologs thereof) found in other varieties of tomato or in other plant species, especially species of the genus *Solanum,* as will be described below. For example, cDNA libraries may be constructed from one or more plant species, one or more varieties, or different tissues of one species or variety. The cDNA libraries may be screened for cysteine synthase cDNAs (using e.g. probes or primers derived from SEQ ID NO: 4 (tomato cysteine synthase cDNA), or fragments or variants thereof). Equally, differential display methods (such as cDNA-AFLP) may be used to identify cysteine synthase transcripts, as described in the Examples. Methods such as TAIL-PCR (Liu et al. 1995, Genomics 25(3):674-81; Liu et al. 2005, Methods Mol Biol. 286:341-8), Linker-PCR, or Inverse PCR (IPCR) may be used to isolate the upstream transcription regulatory region of the gene.

Whether a nucleic acid sequence (or fragment of variant) has constitutive promoter activity, i.e. is capable of conferring constitutive transcription in all organs, whether the activity is "strong.", and whether the activity of the nucleic acid sequence is insensitive to at least one (but preferably more, most preferably any) biotic and/or abiotic stress to which the transgenic cell, tissue, organ or organisms (especially plant or plant cell), may be exposed, can be determined using various methods. Generally, one can distinguish qualitative methods and quantitative methods. Qualitative methods (such as histological GUS staining) are used to determine the spatio-temporal activity of the promoter (is the promoter active or not in a certain tissue or organ, or under certain environmental/developmental conditions?), while quantitative methods (such as fluorometric GUS assays) also quantify the level of activity, compared to controls. Suitable controls are, for example, plants transformed with empty vectors (negative control) or transformed with constructs comprising other promoters, such as CaMV 35S, or non-transgenic plants.

To test and optionally quantify the relative or absolute activity, a cloned or synthetic nucleic acid molecule, such as SEQ ID NO: 1, 2, or variants thereof, or fragments of any of these, may be operably linked to a known nucleic acid sequence (e.g. a reporter gene, such as *gusA,* or any gene encoding a specific protein) and may be used to transform a plant cell using known methods. In some embodiments the cell need not be transformed in a stable manner, i.e. transient expression assays may be used (e.g. protoplast transfection or Agroinfiltration) to determine whether the promoter is active in the cells, tissues or organs and to what degree the promoter is driving transcription. The activity of the promoter can, for example, be assayed (and optionally quantified) by detecting the level of RNA transcripts of the downstream nucleic acid sequence. This may be done using quantitative methods, such as e.g. quantitative RT-PCR or other PCR based methods, and the like. Alternatively, the reporter protein or the activity of the reporter protein may be assayed and quantified. For example, if the reporter gene is the *gus* gene, a fluorometric GUS assay may be used, as described in the Examples. In this way, the quantitative promoter activity levels of transformed plants or plant cells maintained under normal physiological (non-stress) conditions can be compared to levels of plants or plant cells which are exposed to one or more biotic or abiotic stresses. Also, relative or absolute activity levels can be compared to constitutive control promoters, such as the 35S promoter, double-35S promoter, or SEQ ID NO: 1 and/or 2. It is understood that preferably average promoter activity levels are determined and compared using statistical methods.

Thus, in which tissues or organs a promoter according to the invention is active at a certain time (spatio-temporal activity) can, for example, be tested by transforming plants or plant cells with a promoter-reporter gene construct and analyzing various tissues during various developmental stages for the RNA transcript or reporter protein (or its activity). One simple test employs for example histochemical GUS staining, whereby visual assessment of blue colour indicates activity in various tissues and at various developmental stages.

As already mentioned, it is preferred that the promoter activity is constitutive and preferably also strong in plants and plant cells, especially in the host species or variety into which the sequence is introduced. Constitutive activity means that the transcript of any nucleic acid sequence operably linked to the promoter is preferably produced in most tissues (or organs) under most (normal, non-stressed) physiological and developmental conditions. In one embodiment, the promoters according to the invention are at least active in the tissues or organs tested in the Examples, such as leaves (young and old), roots, flowers, seeds, stems (main stem), fruit (e.g. immature and mature fruit), germinated seeds, etc.

Preferably, the promoters according to the invention provide strong, constitutive activity in all plant species, both dicotyledonous species and monocotyledonous species. For example, it was found that SEQ ID NO: 1 and SEQ ID NO: 2 provided strong, constitutive expression in various plant species, such as tomato, tobacco, *Brassica,* melon and lettuce (see Examples).

The strength (quantitative activity) of the AA6 promoters according to the invention (including fragments or variants) in terms of its ability to drive expression of nucleic acid sequences linked downstream (3') can be determined quantitatively using various known methods. For example, the amount of transcribed transcript (mRNA) can be quantified using quantitative RT-PCR or northern blotting. Preferably, the promoter strength is at least essentially equal to that of CaMV 35S (Franck et al., supra) under normal (non-stressed) conditions. "Strong" means, thus, that the promoter strength is preferably at least about identical, but more preferably stronger than that of 35S under normal, non-stressed conditions. Most preferably, the average quantitative promoter activity in the various tissues and organs is at least equivalent to the activity of the CaMV 35S promoter, or is at least 5%, 10%, 20%, 30%, 35%, 40%, 45%, 50%, 55%, 60%, 70%, 75%, or more, higher than the average activity of the CaMV 35S promoter. It is understood that the same copy number and zygosity level of transformants should be compared, e.g. hemizygous or homozygous for the transgene. Preferably, single copy transformants are identified and compared.
The expression of the AA6 promoters remains constitutive and strong under stress conditions, at least under one or more stresses selected from those described elsewhere herein.

Thus, the strength of the promoters according to the invention preferably remains essentially unchanged, or is at least not reduced (or not significantly reduced), when the plant tissues or organs or plants comprising the promoter are exposed to stress conditions, selected at least from one or preferably several of: drought stress, heat stress, water-stress (both too much and too little water), pathogen stress (e.g. virus infection such as CMV, fungal infection, bacterial infection, etc.), pest stress (e.g. insect feeding), wounding, salt stress, radiation stress, etc. Again, quantitative tests can be used to determine this. For example, recombinant plants comprising the promoter may be transferred from a normal temperature environment to a warm environment (such as about 27°C to up to about 50°C), and the promoter activity in various tissues may be compared to the activity in the same tissues under the normal and under the warm temperature conditions.

Variants of the AA6 promoters above also include any isolated nucleic acid promoter of a plant cysteine synthase gene, especially of a cytosolic cysteine synthase gene (i.e. the promoter of a plant gene encoding a cysteine synthase enzyme, especially a cytosolic isoform), which shows strong and constitutive activity in plants and is preferably essentially insensitive to one or more biotic and/or abiotic stresses. As already mentioned, it is likely that also other plant cysteine synthase genes have strong, constitutive promoters, which retain constitutive expression under one or more stress conditions. Plant cysteine synthase genes are genes which encode the enzyme cysteine synthase, also referred to as O-acetyl-L-serine[thiol]-lyase (EC 4.2.99.8). Several cysteine synthase genes have been cloned (see e.g. TC 162833, referred to in the Examples), but the usefulness of their promoters for driving strong, constitutive expression of homologous or heterologous sequences in transgenic plants has not been described. The promoters of such known or as yet unknown cysteine synthase genes may thus be isolated and screened for their activity. For example, cDNA-AFLP, other PCR based methods or Northern hybridization may be used to isolate or identify other cysteine synthase genes, especially those genes which endogenously produced cysteine synthase mRNAs constitutively and also under one or more stress conditions. Those cysteine synthase genes with the desired expression pattern are then selected and their promoter can be cloned using known methods. In a preferred embodiment, the promoter is obtained from a cysteine synthase gene from a plant belonging to the family *Solanaceae,* such as species of the genus *Solanum* (including the reclassified *Lycopersicon* species), *Nicotiana, Capsicum, Petunia, Coffea,* etc.

The promoter according to the invention is preferably not the promoter of a weed plant, such as Arabidopsis thaliana, and is preferably not the promoter of the OASA1 gene of Arabidopsis thaliana as described in Gutierrez-Alcala et al. (J. ofExp. Botany 56, p24872494), or a fragment thereof.

Apart from the cysteine synthase genes provided in SEQ ID NO: 4 (encoding the cysteine synthase enzyme of SEQ ID NO: 5), other cysteine synthase genes can be identified and their promoters isolated. Various methods may be used, as described above.

Thus, in one embodiment of the invention, the promoter of a plant (cytosolic) cysteine synthase gene is described, which has constitutive activity in plants and/or plant cells and whereby the activity is not reduced, or not reduced significantly, when the plant or plant cell, tissue or organ is exposed to one or more biotic and/or abiotic stresses, such as those described.

Such promoters comprise the following: (a) any promoter which is obtainable from a plant and which drives the expression of a plant cysteine synthase gene, especially the promoter of any gene encoding a protein of SEQ ID NO: 5; (b) promoters of those plant genes which encode cysteine synthase enzymes having at least 30, 40, 50, 60, 70, 80, 90, 95, 99%, or more, amino acid identity to SEQ ID NO: 5 (over the entire length); (c) plant promoters driving the expression of nucleic acid sequences, which encode cysteine synthase enzymes, and whereby the nucleic acid sequences comprise at least 30, 40, 50, 60, 70, 80, 90, 95, 98, 99%, or more, nucleotide sequence identity to SEQ ID NO: 4 (over the entire length).

In another embodiment the use of a promoter of a plant cysteine synthase gene for the constitutive over-expression of homologous or heterologous nucleic acid sequences in a recombinant cell or organism, especially a plant cell or plant, is described. This use comprises operably linking the promoter to a homologous or heterologous nucleic acid sequence and transforming a plant or plant cell, as described.

Although the focus above is on the use of the promoters according to the invention in plants and plant cells, the promoters of the invention may also be used for the expression of homologous or heterologous nucleic acid sequences in other cells and organisms, such as in any prokaryotic or eukaryotic cells or organisms, e.g. bacteria, fungi (including yeasts, such as *Pichia, Hansenula,* etc.), mammals, human cells or cell lines, etc.

### Chimeric genes and vectors according to the invention

In one embodiment of the invention any of the above nucleic acid sequences having promoter activity, are used to make chimeric genes, and vectors comprising these for transfer of the chimeric gene into a host cell and expression of an operably linked homologous or heterologous nucleic acid sequence in host cells, such as cells, tissues, organs or whole organisms derived from transformed cell(s).

Host cells are preferably plant cells. Any plant may be a suitable host, such as monocotyledonous plants or dicotyledonous plants, for example maize/corn (Zea species, e.g. *Z. mays, Z. diploperennis* (chapule), *Zea luxurians* (Guatemalan teosinte), *Zea mays* subsp. *huehuetenangensis* (San Antonio Huista teosinte), *Z. mays* subsp. *mexicana* (Mexican teosinte), *Z. mays* subsp. *parviglumis* (Balsas teosinte), *Z. perennis* (perennial teosinte) and *Z. ramosa,* wheat (*Triticum* species), barley (e.g. *Hordeum vulgare*), oat (e.g. *Avena sativa*), sorghum (*Sorghum bicolor*), rye (*Secale cereale*), soybean (*Glycine* spp, e.g. *G. max*), cotton (*Gossypium* species, e.g. *G. hirsutum, G. barbadense*), *Brassica* spp. (e.g. *B. napus, B. juncea, B. oleracea, B. rapa,* etc.), sunflower (*Helianthus annus*), tobacco (*Nicotiana* species), alfalfa (*Medicago sativa*), rice (*Oryza* species, e.g. *O. sativa indica* cultivar-group or *japonica* cultivar-group), forage grasses, pearl millet (*Pennisetum* species. e.g. *P. glaucum*), tree species, vegetable species, such as *Lycopersicon* ssp (recently reclassified as belonging to the genus *Solanum*), e.g. tomato (*L. esculentum,* syn. *Solanum lycopersicum*) such as e.g. cherry tomato, var. *cerasifonne* or current tomato, var. *pimpinellifolium*) or tree tomato (*S. betaceum,* syn. *Cyphomandra betaceae*), potato (*Solanum tuberosum*) and other *Solanum* species, such as eggplant (*Solanum melongena*), pepino (*S. muricatum*), cocona (*S. sessiliflorum*) and naranjilla (*S. quitoense*); peppers (*Capsicum annuum, Capsicum frutescens*), pea (e.g. *Pisum sativum*), bean (e.g. *Phaseolus* species), carrot (*Daucus carota*), *Lactuca* species (such as *Lactuca sativa, Lactuca indica, Lactuca perennis*), cucumber (*Cucumis sativus*), melon (*Cucumis melo*), zucchini (*Cucurbita pepo*), squash (*Cucurbita maxima, Cucurbita pepo, Cucurbita mixta*), pumpkin (*Cucurbita pepo*), watermelon (*Citrullus lanatus* syn. *Citrullus vulgaris*), fleshy fruit species (grapes, peaches, plums, strawberry, mango, melon), ornamental species (e.g. Rose, *Petunia, Chrysanthemum,* Lily, Tulip, *Gerbera* species), woody trees (e.g. species of *Populus, Salix, Quercus, Eucalyptus*), fibre species e.g. flax (*Linum usitatissimum*) and hemp (*Cannabis sativa*). In one embodiment vegetable species, especially *Solanum* species (including *Lycopersicon* species) are preferred.

Thus, for example species of the following genera may be transformed: *Cucurbita, Rosa, Vitis, Juglans, Fragaria, Lotus, Medicago, Onoblychis, Trifolium, Trigonella, Vigna, Citrus, Linum, Geranium, Manihot, Daucus, Arabidopsis, Brassica, Raphanus, Sinapis, Atropa, Capsicum, Datura, Cucumis, Hyoscyanus, Lycopersicon, Solanum, Nicotiana, Malus, Petunia, Digitalis, Majorana, Ciahorium, Helianthus, Lactuca, Bromus, Cittullus, Asparagus, Antirrhinum, Heterocallis, Nemesis, Pelargonium, Panieum, Pennisetum, Ranunculus, Senecio, Salpiglossis, Browaalia, Glycine, Pisum, Phaseolus, Gossypium, Glycine, Lolium, Festuca, Agrostis.* A further preference is for each of *Cucurbita, Brassica, Lycopersicon, Solanum, Oryza* and *Zea.* A preference is for each of *Avena, Medicago, Capsicium, Nicotiana, Lactuca, Pisum, Cucumis, Cucurbita, Brassica, Solanum* (including *Lycopersicon*), *Oryza* and *Zea.*

The construction of chimeric genes, and vectors for introduction of chimeric genes into the genome of host cells, is generally known in the art. To generate a chimeric gene the AA6 promoter sequence is operably linked to another nucleic acid sequence which is to be transcribed in the host cells, using standard molecular biology techniques. The promoter sequence may already be present in a vector so that the nucleic acid sequence which is to be transcribed is simply inserted into the vector downstream of the promoter sequence. The vector is then used to transform the host cells and the chimeric gene is preferably inserted in the nuclear genome or into the plastid, mitochondrial or chloroplast genome, so that the downstream nucleic acid sequence is expressed due to the activity of the promoter (e. g., Mc Bride et al., 1995 Bio/Technology 13, 362; US 5,693, 507).

A chimeric gene, therefore, preferably comprises an AA6 promoter as described above, operably linked to a homologous or heterologous nucleic acid sequence, and optionally followed by a 3' nontranslated nucleic acid sequence (3'UTR). The homologous or heterologous nucleic acid sequence may be a sequence encoding a protein or peptide, or it may be a sequence which is transcribed into an active RNA molecule, such as an sense and/or antisense RNA (dsRNA) suitable for silencing a gene or gene family in the host cell or organism.

The AA6 promoter-comprising chimeric gene can be stably inserted in a conventional manner into the nuclear genome of a single plant cell, and the so-transformed plant cell can be used in a conventional manner to produce a transformed plant that has an altered phenotype due to the constitutive expression of the chimeric gene.

In this regard, a T-DNA vector, comprising an AA6 promoter (or variant or fragment as described above) operably linked to a further nucleic acid sequence, in *Agrobacterium tumefaciens* can be used to transform the plant cell, and thereafter, a transformed plant can be regenerated from the transformed plant cell using the procedures described, for example, in EP 0 116 718, EP 0 270 822, PCT publication WO 84/02913 and published European Patent application EP 0 242 246 and in Gould et al. (1991, Plant Physiol. 95,426-434). The construction of a T-DNA vector for *Agrobacterium* mediated plant Transformation is well known in the art. The T-DNA vector may be either a binary vector as described in EP 0 120 561 and EP 0 120 515 or a co-integrate vector which can integrate into the *Agrobacterium* Ti-plasmid by homologous recombination, as described in EP 0 116 718.

Preferred T-DNA vectors each contain an AA6 promoter operably linked to the nucleic acid sequence to be transcribed between T-DNA border sequences, or at least located to the left of the right border sequence. Border sequences are described in Gielen et al. (1984, EMBO J 3,835-845). Of course, other types of vectors can be used to transform the plant cell, using procedures such as direct gene transfer (as described, for example in EP 0 223 247, or particle or microprojectile bombardment as described in US 2005/055740 and WO 2004/092345), pollen mediated transformation (as described, for example in EP 0 270 356 and WO 85/01856), protoplast transformation as, for example, described in US 4,684, 611, plant virus- mediated Transformation, liposome-mediated Transformation (as described, for example in US 4,536, 475), and other methods such as those described methods for transforming certain lines of maize (e. g., US 6,140, 553; Fromm et al., 1990, Bio/Technology 8, 833-839; Gordon-Kamm et al., 1990, The Plant Cell 2, 603-618) and rice (Shimamoto et al., 1989, Nature 338, 274-276; Datta et al. 1990, Bio/Technology 8, 736-740) and the method for transforming monocots generally (WO 92/09696). For cotton Transformation see also WO 00/71733, and for rice Transformation see also the methods described in WO 92/09696, WO 94/00977 and WO 95/06722. For sorghum Transformation see e.g. Jeoung JM et al. 2002, Hereditas 137: 20-8 or Zhao ZY et al. 2000, Plant Mol Biol. 44:789-98). For tomato or tobacco Transformation see also An G. et al., 1986, Plant Physiol. 81: 301-305; Horsch R.B. et al., 1988, In: Plant Molecular Biology Manual A5, Dordrecht, Netherlands, Kluwer Academic Publishers. pp 1-9; Koornneef M. et al., 1986, In: Nevins D.J. and R.A. Jones, eds. Tomato Biotechnology, New York, NY, USA, Alan R. Liss, Inc. pp 169-178). Likewise, selection and regeneration of transformed plants from transformed cells is well known in the art. Obviously, for different species and even for different varieties or cultivars of a single species, protocols are specifically adapted for regenerating transformants at high frequency.

Besides transformation of the nuclear genome, also Transformation of the plastid genome, preferably the chloroplast genome, is included in the invention. One advantage of plastid genome Transformation is that the risk of spread of the transgene(s) can be reduced. Plastid genome transformation can be carried out as known in the art, see e.g. Sidorov VA et al. 1999, Plant J.19: 209-216 or Lutz KA et al. 2004, Plant J. 37(6):906-13.

The resulting transformed plant can be used in a conventional plant breeding scheme to produce more transformed plants containing the transgene. Single copy transformants can be selected, using e.g. Southern Blot analysis or PCR based methods or the Invader® Technology assay (Third Wave Technologies, Inc.). Transformed cells and plants can easily be distinguished from non-transformed ones by the presence of the chimeric gene. The sequences of the plant DNA flanking the insertion site of the transgene can also be sequenced, whereby an "Event specific" detection method can be developed, for routine use. See for example WO 01/41558, which describes elite event detection kits (such as PCR detection kits) based for example on the integrated sequence and the flanking (genomic) sequence.

In one embodiment the nucleic acid sequence which is to be transcribed, and optionally translated (if it is a coding sequence), is inserted into the plant genome so that the sequence to be transcribed is upstream (i.e. 5') of suitable 3'end transcription regulation signals ("3'end") (i.e. transcript formation and polyadenylation signals). Polyadenylation and transcript formation signals include those of the nopaline synthase gene ("3' nos") (Depicker et al., 1982 J. Molec. Appl. Genetics 1, 561-573.), the octopine synthase gene ("3'ocs") (Gielen et al., 1984, EMBO J 3, 835-845) and the T-DNA gene 7 ("3' gene 7") (Velten and Schell, 1985, Nucleic Acids Research 13, 6981-6998), which act as 3'-untranslated DNA sequences in transformed plant cells, and others.

In a preferred embodiment the 3'end sequence used is that of a plant cysteine synthase gene, preferably from the cysteine synthase gene from which also the AA6 promoter is obtainable. Suitable 3'end sequences provided herein are SEQ ID NO: 3, or a fragment or variant thereof. A variant of SEQ ID NO: 3 includes nucleic acid sequences comprising at least 50, 60, 65, 70, 75, 80, 85, 90, 95, 98, 99%, or more, nucleic acid sequence identity to SEQ ID NO: 3. Fragments of SEQ ID NO: 3, or of variants of SEQ ID NO: 3, include nucleic acid sequences comprising at least 50, 100, 150, 200, 250, 300 or more consecutive nucleotides of SEQ ID NO: 3 or of a variant of SEQ ID NO: 3. These 3'end sequences are also an embodiment as such, and may be used to construct any chimeric gene and vector, i.e. also in combination with a different promoter.

The nucleic acid sequence to be expressed is in one embodiment a sequence encoding a protein or peptide, including hybrid proteins or peptides or fusion proteins. The coding sequence may be of any origin, i.e. plant, fungus (including yeast), animal, bacterial, synthetic, viral, etc. It may also comprise a sequence encoding a targeting peptide, such as a secretion signal peptide or a plastid targeting signal. A coding sequence may also be linked in-frame to a gene encoding a selectable or scorable marker, such as for example the neo (or nptII) gene (EP 0 242 236) conferring kanamycin resistance, so that the cell expresses a fusion protein which is easily detectable. Although the coding region (cDNA or genomic DNA) of any gene may be used, examples of the coding regions of the following genes are preferably operably linked to an AA6 promoter according to the invention: 1. inverted repeat sequences of viral nucleic acid sequences (e.g. sense and antisense sequences of viral coat protein genes; see also below); 2. disease signal transduction pathway genes or disease resistance genes; 3. abiotic stress response related genes (e.g. SHINE transcription factors, or CBF/DREB genes); 4. secondary metabolite biosynthesis genes, including genes for the production of therapeutic and/or pharmacologically important products or industrially valuable compounds.

Obviously, also other genes may be operably linked to the AA6 promoters of the invention, such as genes affecting agronomic traits, such as genes for herbicide resistance (e.g. EPSPS genes, the bar or PAT gene), genes affecting yield or quality traits (e.g. protein composition), and the like.

The chimeric genes or vectors according to the invention can also be used to transform microorganisms, such as bacteria (e.g. *Escherichia coli, Pseudomonas, Agrobacterium, Bacillus,* etc.) or fungi or algae or insects, or the genes or vectors may be used to engineer viruses. Transformation of bacteria with nucleic acid sequence of this invention, incorporated in a suitable cloning vehicle, can be carried out in a conventional manner, preferably using conventional electroporation techniques as described in Maillon et al. (1989, FEMS Microbiol. Letters 60, 205-210.) and WO 90/06999. For expression of coding sequences in prokaryotic host cell, the codon usage of the nucleic acid sequence may be optimized accordingly (likewise, for expression of coding sequences in cells, codon usage of the nucleic acid sequence may be optimized as known). Intron sequences should be removed and other adaptations for optimal expression may be made as known.

For obtaining enhanced expression of a nucleic acid sequence in monocot plants such as grass species, e.g. maize or rice, an intron, preferably a monocot intron, can be added to the chimeric gene. For example the insertion of the intron of the maize Adhl gene into the 5' regulatory region has been shown to enhance expression in maize (Callis et. al., 1987, Genes Develop. 1: 1183-1200). Likewise, the HSP70 intron, as described in US 5,859, 347, may be used to enhance expression. Thus, one or more introns may optionally be inserted into any of the promoter sequences according to the invention, or into the 5' UTR or the coding sequence.

In another embodiment the AA6 promoter is used to make a chimeric gene and vector for gene silencing, whereby the promoter is operably linked to a sense and/or antisense nucleic acid sequence of a target gene (endogenous gene or gene family which is to be silenced). In yet another embodiment the target gene may also be a gene or gene family of an invading plant pathogen, such as a virus. For example, an inverted repeat sequence of a viral coat protein gene may be used for making virus resistant plants. Viral coat protein genes are for example described in WO 96/21031. "Gene silencing" refers to the down-regulation or complete inhibition of gene expression of one or more target genes. The use of inhibitory RNA to reduce or abolish gene expression is well established in the art and is the subject of several reviews (e.g. Baulcombe, 1996, Plant Cell 8: 1833-1844; Stam et al., 1997, Plant Journal 12: 63-82; Depicker and Van Montagu, 1997, Curr. Opinion Cell Biol. 9: 373-382). There are a number of technologies available to achieve gene silencing in plants, such as chimeric genes which produce antisense RNA of all or part of the target gene (see e.g. EP 0 140 308 B1, EP 0 240 208 B1 and EP 0 223 399 B1), or which produce sense RNA (also referred to as co-suppression), see EP 0 465 572 B1.

The most successful approach so far has however been the production of both sense and antisense RNA of the target gene ("inverted repeats"), which forms double stranded RNA (dsRNA) in the cell and silences the target gene(s). Methods and vectors for dsRNA production and gene silencing have been described in EP 1 068 311, EP 983 370 A1, EP 1 042 462 A1, EP 1 071 762 A1 and EP 1 080 208 A1.

A vector according to the invention may therefore comprise an AA6 promoter operably linked to a sense and/or antisense DNA fragment of a target gene. Short (sense and antisense) stretches of the target gene sequence, such as at least about 17, 18, 19, 20, 21, 22, 23, 24 or 25 nucleotides of coding or non-coding sequence may be sufficient. Longer sequences are frequently also used, such as at least about 100, 200, 250, 300, 400, 500, 1000, 1500 nucleotides, or more. Preferably, the sense and antisense fragments are separated by a spacer sequence, such as an intron, which forms a loop (or hairpin) upon dsRNA formation. Any stretch of the target gene may be used to make a gene silencing vector and a transgenic plant in which the target gene or gene family is silenced. A convenient way of generating hairpin constructs is to use generic vectors such as pHANNIBAL and pHELLSGATE, vectors based on the Gateway^{®} technology (see Wesley et al. 2004, Methods Mol Biol. 265:117-30; Wesley et al. 2003, Methods Mol Biol. 236:273-86 and Helliwell & Waterhouse 2003, Methods 30(4):289-95.), all incorporated herein by reference.

By choosing conserved nucleic acid sequences of the target gene, the family members in a host plant can be silenced. Where the target gene is a gene or gene family of an invading pathogen, the pathogen's target **gene** will be silenced and the plant will become resistant to the pathogen. Encompassed herein are also transgenic plants comprising an AA6 promoter, operably linked to a sense and/or antisense DNA fragment of a target gene nucleic acid sequence and exhibiting a target gene silencing phenotype. The phenotype will depend on the function of the gene, and may be a chemical or molecular change, macroscopically visible or not visible. Such chimeric genes and vectors can, therefore, also be used to determine or verify the function of genes.

The chimeric genes according to the invention may be introduced stably into the host genome or may be present as an episomal unit.

### Transgenic cells and organisms according to the invention

Transgenic cells and organisms, especially plants, plant cells, tissues or organs are provided, obtainable by the above methods. These cells and organisms are characterized by the presence of a chimeric gene in their cells or genome by the presence of an AA6 promoter according to the invention. In addition, the mRNA transcript or the translated protein, may alter the phenotype of the cells or organism, e.g. of the plant cell or plant.

Although the AA6 promoter is constitutive, the position in the genome can affect the activity of the promoter and the expression level of the chimeric gene. Therefore, transformants ("Events" or "Transformation Events") expressing high, constitutive levels of the protein or of the sense and/or antisense transcript (when silencing constructs are used) can be selected by e.g. analysing copy number (Southern blot analysis), mRNA transcript levels (e.g. Northern blot analysis or RT-PCR) or by analysing the presence and level of protein encoded by the nucleic acid sequence (e.g. SDS-PAGE followed by Western blot analysis; ELISA assays, immunocytological assays, etc). The transformants can also be tested for the stability of expression under one or more biotic and/or abiotic stress conditions and those events which retain high, constitutive expression under one or more of the desired conditions can be identified and selected for further use.

The transgenic plants can be used in traditional breeding methods, such as crossing, selfing, backcrossing, etc. By selfing the transformants, plants which are homozygous for the transgene can be generated. Breeding procedures are known in the art and are described in standard text books of plant breeding, e.g., Allard, R.W., Principles of Plant Breeding (1960) New York, NY, Wiley, pp 485; Simmonds, N.W., Principles of Crop Improvement (1979), London, UK, Longman, pp 408; Sneep, J. et al., (1979) Tomato Breeding (p. 135-171) in: Breeding of Vegetable Crops, Mark J. Basset, (1986, editor), The Tomato crop: a scientific basis for improvement, by Atherton, J.G. & J. Rudich (editors), Plant Breeding Perspectives (1986); Fehr, Principles of Cultivar Development—Theory and Technique (1987) New York, NY, MacMillan.

Transgenic cells or organisms can also be used in cell cultures (plant cell cultures, bacterial or fungal cell cultures such as yeast cultures, human or mammalian cell cultures, insect cell cultures), for example for the large scale production of recombinant proteins. In one embodiment a cell culture is provided, comprising cells comprising an AA6 promoter according to the invention.

### Methods and uses according to the invention

Also disclosed is a method for making a transgenic plant or plant cell, comprising the steps of:
(a) generating a chimeric gene or a vector comprising an AA6 promoter and/or an AA6 3'UTR according to the invention, operably linked to a nucleic acid sequence to be expressed;
(b) transforming a plant or plant cell with said chimeric gene or vector; and, optionally,
(c) regenerating a transgenic plant or plants.
Further, transgenic plants may be identified which provide strong, constitutive promoter activity under non-stress conditions, and whereby the promoter activity remains essentially unchanged (is at least not reduced, or not reduced significantly) when the plant is exposed to one or more biotic and/or abiotic stresses.

The plants may be used in conventional agricultural and breeding methods. In particular, they may be grown in environments which subject the plants to one or more biotic and/or abiotic stress conditions, without reducing the activity of the promoter. Thus, a method for growing transgenic plants in e.g areas with high or low temperature, strong winds, high salt, high soil contaminants, high or low water levels, drought spells, areas of high disease or pest pressure, high radiation, etc. without reducing, or at least without significantly reducing the promoter activity is provided.

### SEQUENCES

SEQ ID NO 1: "3kb" (2986 bp) AA6 promoter
SEQ ID NO 2: "5kb" (5000 bp) AA6 promoter
SEQ ID NO 3: 3'UTR of tomato cysteine synthase gene (AA6 gene)
SEQ ID NO 4: tomato cysteine synthase cDNA (AA6 cDNA)
SEQ ID NO 5: protein encoded by SEQ ID NO: 4 (tomato cytosolic cysteine synthase)
SEQ ID NO 6: "3kb" AA6 promoter with some ambiguities
SEQ ID NO 7: "5kb" AA6 promoter with some ambiguities
SEQ ID NO 8: "3kb" AA6 promoter of pKG8135, as re-sequenced and as in E. coli deposit CBS120175)
SEQ ID NO 9: "5kb" AA6 promoter of pKG8137, as re-sequenced and as in E. coli deposit CBS120176)

### Figures

- Figure 1:: Vector map of pKG1562
- Figure 2:: Vector map of pKG1700
- Figure 3:: Vector map of pKG8135, pKG8136 and pKG8137.

The following non-limiting Examples describe the use of AA6 promoters according to the invention. Unless stated otherwise in the Examples, all recombinant DNA techniques are carried out according to standard protocols as described in Sambrook et al. (1989) Molecular Cloning: A Laboratory Manual, Second Edition, Cold Spring Harbor Laboratory Press, and Sambrook and Russell (2001) Molecular Cloning: A Laboratory Manual, Third Edition, Cold Spring Harbor Laboratory Press, NY; and in Volumes I and 2 of Ausubel et al. (1994) Current Protocols in Molecular Biology, Current Protocols, USA. Standard materials and methods for plant molecular work are described in Plant Molecular Biology Labfax (1993) by R.D.D. Croy, jointly published by BIOS Scientific Publications Ltd (UK) and Blackwell Scientific Publications, UK.

### EXAMPLES

### Example 1 - Materials and Methods

Differential display analysis using cDNA was performed on *Lycopersicon* esculentum var. Moneyberg.
Testing expression under stress conditions was performed on *L.* esculentum var. Moneyberg.
Transformations using vectors pKG8136, pKG8137 and pKG1700 were performed in *Nicotiana tabacum* var. SRI and *L.* esculentum var. RZ 52201. Plant transformation was carried out as described in the following references:
For tomato and tobacco transformation:
   An G., B.D. Watson and C.C. Chiang. 1986. Transformation of tobacco, tomato, potato, and Arabidopsis thaliana using a binary Ti vector system. Plant Physiol. 81: 301-305.
For tobacco Transformation: Horsch R.B., J. Fry, N. Hoffman, J. Neidermeyer, S.G. Rogers and R.T. Fraley. 1988. Leaf disc transformation. In: Plant Molecular Biology Manual A5. Dordrecht, Netherlands, Kluwer Academic Publishers. pp 1-9.
For tomato transformation: Koornneef M., M. Jongsma, R. Weide, P. Zabel and J. Hille. 1986. Transformation of tomato. In: Nevins D.J. and R.A. Jones, eds. Tomato Biotechnology. New York, NY, USA, Alan R. Liss, Inc. pp 169-178.

5' and 3' end RACE was performed using the SMART™ RACE cDNA Amplification Kit from CLONTECH Laboratories Inc.

Cloning of PCR products was performed with the Original TA Cloning® Kit from Invitrogen BV. using plasmid pCR®2.1.

DNA sequencing was performed by BaseClear (P.O. Box 1336, 2302 BH, Leiden, The Netherlands).

Transformation vectors were incorporated into *Agrobacterium tumefaciens* var. GV2260.

Qualitative analysis of promoter activity was carried out using histological GUS assays: Various plant parts were incubated overnight at 37°C with shaking in the presence of atmospheric oxygen with Xgluc substrate in phosphate buffer (5-bromo-4-chloro-indolyl glucuronide, 1 mg/ml, K₂HPO₄, 40 mM, KH₂PO₄, 10 mM, pH 7.4). The samples were de-stained by repeated washing with ethanol. Non-transgenic plants were used as negative controls.

Quantitative analysis of promoter activity was carried out using a fluorometric GUS assay:
Total protein samples were prepared from young leaf material; samples were prepared from pooled leaf pieces of approximately the same size and developmental stage from different parts of each plant tested. Fresh leaf material was ground in phosphate buffer (Na₂HPO₄, 77.4 mM, NaH₂PO₄, 22.6 mM) using metal beads followed by centrifugation and collection of the supernatant.

### Fluorometric GUS assays:

Protein concentration in an aliquot from each supernatant was measured using the Nano Orange Kit from Molecular Probes, Inc. Protein samples were diluted to normalize the total protein concentration. Aliquots of the protein samples were incubated overnight at 37°C with the substrate 4-methyl umbelliferyl β-d-glucuronide (MUG) (end concentration 1mg/ml). Fluorescence measurements at time zero and after overnight incubation were taken by removing aliquots of the reaction mixture and adding Na₂CO₃ solution (end concentration 1.1M) to stop the reaction then measuring emission at 460nm caused by excitation at 355nm.

### Example 2 - Selection of a gene for promoter isolation

Differential analysis was performed using cDNA-AFLP® (Volkmuth W., et al., 2003, Genome-Wide cDNA-AFLP® Analysis of the Arabidopsis Transcriptome, OMICS, 7, 2; Vos, P. and Stanssens P., 2002, AFLP-based transcript profiling, Current Protocols in Molecular Biology, unit 25B.5., Ausubel, F.M., Brent, R., Kingston, R.E., Moore, D.D., Seidman, J.G. Smith, J.A., Struhl, K., John Wiley and Sons, New York; and Vos et al., 1995, AFLP: a new technique for DNA fingerprinting, Nucleic Acids Research 23: 4407-4414) on cDNA isolated from tomato plant. material at various stages of development (young leaf, old leaf, stem, root, *in vitro* leaf, *in vitro* stem, *in vitro* root, callus, immature and mature green fruit, breaker stage fruit, red fruit, flowers, total seedlings). Expression profiles were produced using all possible Taq1/Mse1 primer combinations with two selective nucleotides for the Taq1 primer and three selective nucleotides for the Mse1 primer. Subsequent gene fragment expression profiles were produced using Keygene N.V. proprietary software (Improve^{™}) and stored in a database. Thirteen candidate transcripts corresponding to 12 Taq1/Mse1 primer combinations were selected which showed strong, constitutive expression in all sampled tissues.
Expression of the candidate genes was then measured by cDNA-AFLP® in various tissues under biotic and abiotic stresses. Three week old tomato plants (Moneyberg) were grown for 14 days with the following separate stress conditions:
infection with Cucumber Mosaic Virus (CMV), high temperature (37°C day and 25°C night), low temperature (10°C day and 5°C night), drought stress (minimal water to avoid plant death, with 37°C day and 25°C night) and wounding (crushing leaves with tweezers) 2 days prior to harvest.

The following material was harvested: young leaves, old leaves, stem, roots and flowerbuds. cDNA from these materials was produced and differential analysis performed using cDNA-AFLP® with the same primer combinations (and also using primer combinations with fewer selective nucleotides) required to amplify the 13 selected candidate transcripts (see below for primer sequences). Seven transcripts were selected which continued to give strong, constitutive expression under all the above stress conditions.

The primer sequences of the (+2/+2) AFLP primers which amplify the cysteine synthase gene (AA6) fragment are:

| AFLP primer code | Keygene code | Sequence |
|---|---|---|
| Tr13 | 96F06 | 5' GTAGACTGCGTACCGAAG 3' |
| M26 | 92N16 | 5' GATGAGTCCTGAGTAATT 3' |

The candidate cDNA AFLP fragments were cut from the gel, purified, cloned and sequenced. The sequence information was then used to design primers for RACE PCR.

Both 5' and 3' end SMART-RACE-PCR (Clontech) was performed to produce extended gene fragments of the 7 candidate genes, these were cloned into pCR2.1 (Invitrogen) and subsequently sequenced (BaseClear, P.O. Box 1336, 2302 BH, Leiden, The Netherlands). The extended gene fragments were assembled along with homologous ESTs from public databases. This resulted in full length cDNA sequences for 5 candidate genes.

A copy count assessment for the 5 genes was performed using Southern blotting with tomato genomic DNA digested with 10 different restriction enzymes and using 5' end fragments of the cDNAs as probes. The same probes were also hybridized to an existing Moneyberg BAC library (Keygene N.V.).

One of the full length cDNAs (designated IS158-53; SEQ ID NO: 4) showed very high homology to the tomato cytosolic cysteine synthase gene (tomato|TC162833 homologue to UP|Q9FS27 (Q9FS27). TC162833 and SEQ ID NO: 4 both encode the same amino acid sequence (SEQ ID NO: 5), but differ at the nucleic acid level in two nucleotides. Nucleotide 'C' at position 207 of SEQ ID NO: 4 is a 'T' in TC162833, while nucleotide 'A' at position 372 of SEQ ID NO: 4 is a 'G' in TC162833.

SEQ ID NO: 4 was expressed constitutively even under biotic and abiotic stress conditions, it was present in low copy number (approximately two copies) in the tomato genome and it hybridized well to BAC 9 of the Keygene N.V. tomato BAC library, therefore isolation of its promoter was undertaken.

The promoter of the gene whose full length cDNA has homology with the tomato cysteine synthase gene (SEQ ID NO: 4) was selected, as described below.

### Example 3 - AA6 promoter sequence isolation

Primers designed on the derived sequence of the tomato gene designated as cysteine synthase (see below for primer sequences) were used to perform linker PCR using genomic DNA from BAC 9 as template. Both 5' and 3' sequence was obtained for the UTRs of cysteine synthase. The 3' UTR sequence (SEQ ID NO: 3) was obtained by long range PCR using genomic DNA of Moneyberg tomato as template.

Primers designed on cysteine synthase sequence:
For promoter 5'-GTTCGATGAGGACACTCTCGC-3
   and nested primer 5'-CAATTAAAGTTGCTAAGCGTCCTGA-3'
For terminater 5'-TCAGTTACATCCTTGGCAATTCC-3'
   and nested primer 5'-CAGAGAACATGACTGTGGAGCC-3'

### Example 4 - Transformation vector construction

Two AA6 promoter DNA fragments, one of 5000bp (SEQ ID NO: 2) and one of 2986bp (SEQ ID NO: 1), of the cysteine synthase 5' upstream region were ligated, together with the *gusA* coding region, into plasmid the pKG1562 (vector backbone, containing nptII driven by the nopaline synthase promoter) with either the endogenous cysteine synthase terminator (SEQ ID NO: 3) or the *nos* terminator.

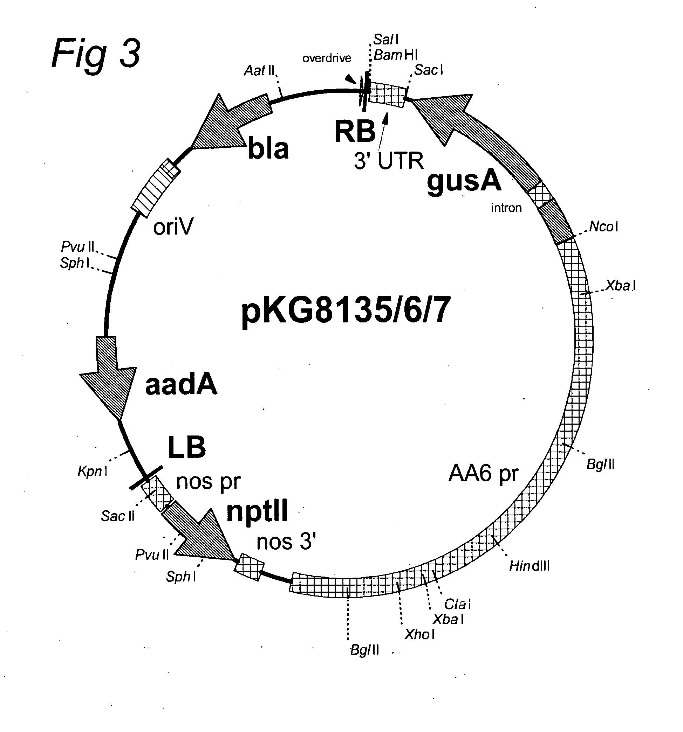

The constructs were designated:
pKG8135 containing (see Fig. 3):
   2986 bp of the 5'upstream region of cysteine synthase (SEQ ID NO: 1): *gusA:* 3'*nos*
pKG8136 containing (see Fig. 3):
   5000 bp of the 5'upstream region of cysteine synthase (SEQ ID NO: 2): *gusA: 3'nos*
pKG8137 containing (see Fig. 3):
   5000 bp of the 5'upstream region of cysteine synthase (SEQ ID NO: 2): *gusA:* 3'cysteine synthase (SEQ ID NO: 3)

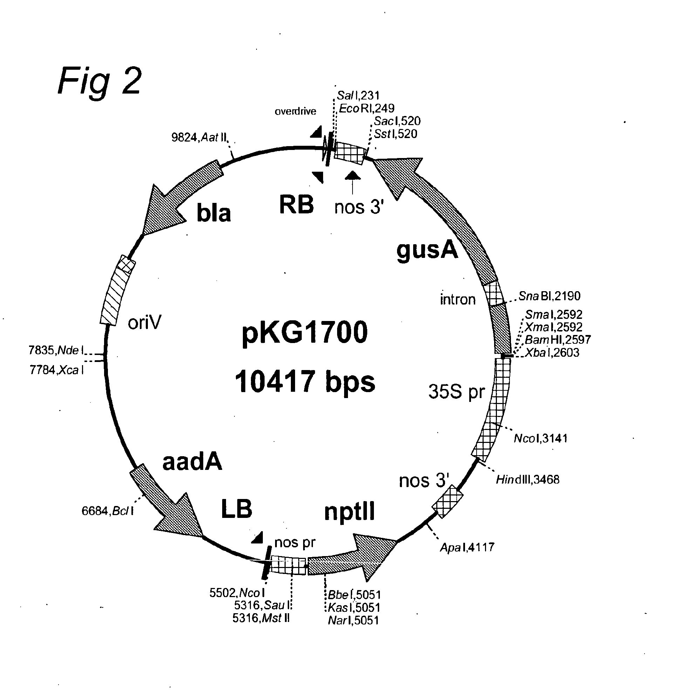

A control construct was used, as follows (see Fig. 2):
pKG 1700 containing: CaMV35S promoter (Franck *et al.*, supra): *gusA :* 3' *nos*

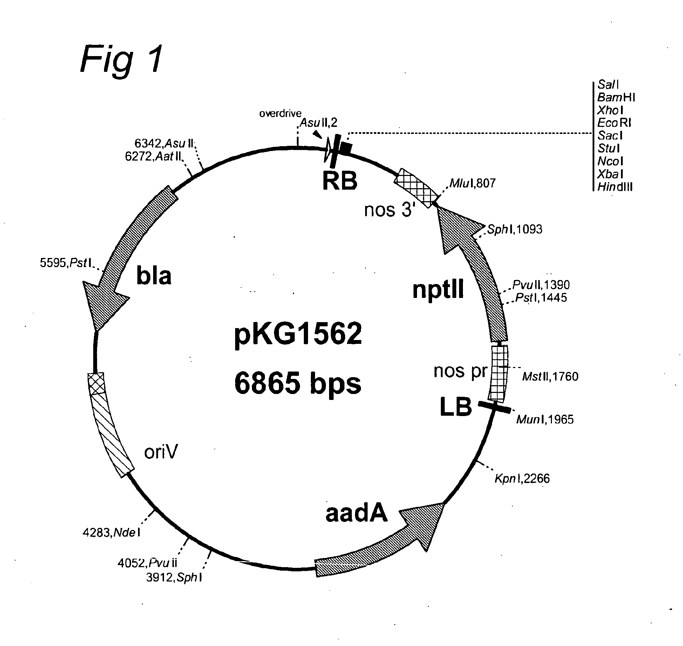

See Figures 1-3 for vector map details of transformation vectors used.

The plasmids pKG8135, pKG8136, pKG8137 and pKG1700 were incorporated into *Agrobacterium tumefaciens.* Tobacco (var. SR1) and tomato (var. RZ 52201) were transformed and regenerated under kanamycin selection and primary regenerants (To) were grown to seed.

### Example 5 - Expression analysis of transfomiants

Since expression of *gusA* is driven by the AA6 promoter, or the CaMV35S promoter, in these vectors, the level of β-D-glucuronidase (GUS) produced in the plants indicates the effectiveness of the promoter. Histological staining assays for the presence of GUS were performed using 5-bromo-4-chloro-indolyl glucuronide (XGluc) as substrate.

Staining experiments were performed on leaf tissue of both tobacco and tomato T1 plants (obtained after selfing of primary transformants) at several stages of development from seedlings through to flowering plants. Additionally, histological staining experiments for GUS were performed on a range of material from mature tobacco and tomato lines, including flower buds, open flowers, flower stems (tobacco), flower stalks, apical meristem buds, leaf, leaf stalks, main stem, roots, seeds and fruit (tomato) and also on germinating seeds and seedlings.

Expression of GUS was detected in all tissues tested in both tobacco and tomato when *gusA* was driven by either the long fragment (SEQ ID NO: 2) or short fragment (SEQ ID NO: 1) of the AA6 promoter or the CaMV35S promoter.

Visual (color staining) comparison indicated that the AA6 promoter fragments performed as well as the CaMV35S promoter in both tobacco and tomato.

Quantitative analysis of GUS expression was performed with a fluorometric assay using 4-methyl umbelliferyl β-d-glucuronide (MUG) as substrate. Expression of GUS driven by the 5000bp fragment AA6 promoter (pKG8137) was compared with expression of GUS driven by the CaMV35S promoter (pKG1700) in both tobacco and tomato young leaves.

Single copy plants were selected using Southern blotting; genomic DNA from the tobacco and tomato plants which was digested with HindIII restriction enzyme and an nptII fragment was used as probe, the process was repeated using XbaI as restriction enzyme. Since each T-DNA insert contains one copy each of nptII and gusA, the nptII copy number reflects the *gusA* copy number. Copy numbers were also confirmed using an Invader® assay (Third Wave Technologies, Inc.) detecting the presence and relative quantity of the nptII gene in comparison to a known tomato or tobacco internal control; application of this assay on the T1 generation allows discrimination between hemizygous and homozygous individuals.

Total protein extracts were made from pKG8137 and pKG1700 hemizygous and homozygous single copy transformants and zero copy controls of tobacco and tomato plants. Protein content was measured using the NanoOrange^{™} Kit (Molecular Probes) and then protein concentration in the samples was normalized by dilution with extraction buffer.

After addition of MUG substrate the initial fluorescence was measured (excitation filter, 355nm and emission filter, 460nm). Samples were then incubated and fluorescence due to cleaved methyl umbelliferon (MU) was measured. Increase in fluorescence per hour of incubation was calculated. Calibration curves showing GUS enzyme (Sigma-Aldrich Chemie B.V.) activity in a background of non-transgenic tobacco or tomato total protein extract (equal to the total protein content of the test samples) were produced. Concentrations of GUS enzyme (mU/mg of total plant protein extract) present in the transgenic test samples were calculated using the fluorescence data from the samples and calibration curves.

Statistical analysis of duplicate experiments indicated that the levels of GUS expression driven by the AA6 promoter (pKG8137) and the CaMV35S promoter were not significantly different in tobacco, whereas in tomato, expression of GUS due to the AA6 promoter was up to 79% higher than due to the CaMV35S promoter, in homozygous single copy plants (data sets shown in Tables below).

### Expression of GUS in tobacco

**Table 1: Average GUS activity (expressed in mU/mg of total plant protein extract) per line for hemizygous tobacco plants with their standard deviation and standard error of means (SEM).**

| Line / Promoter | data set 1 | | | data set 2 | | |
|---|---|---|---|---|---|---|
| line | average | standard deviation | SEM | average | standard deviation | SEM |
| AA6-03 | 14250 | 9702 | 4339 | 26471 | 20251 | 9057 |
| AA6-05 | 4107 | 1773 | 793 | 5411 | 3021 | 1511 |
| AA6-06 | 6785 | 3965 | 1773 | 13608 | 7701 | 3144 |
| AA6-07 | 3451 | 1600 | 716 | 3323 | 1403 | 627 |
| | | | | | | |
| 35S-06 | 5151 | 2535 | 1268 | 10294 | 6224 | 2783 |
| 35S-09 | 5316 | 2105 | 1052 | 9303 | 2745 | 1228 |
| 35S-13 | 15557 | 18869 | 9435 | 21330 | 12911 | 5774 |
| 35S-25 | 11226 | 10219 | 5110 | 15114 | 7978 | 3568 |
| 35S-28 | 1425 | 23 | 10 | 1388 | 10 | 4 |
| 35S-29 | 8796 | 4370 | 1954 | 20911 | 8540 | 3819 |
| neg control | 1425 | 23 | 10 | 1388 | 10 | 4 |
| average AA6 | 7148 | 5375 | 2687 | 12203 | 11074 | 5537 |
| average 35S | 7912 | 8728 | 3563 | 13057 | 7633 | 3116 |

**Table 2: Average GUS activity (expressed in mU/mg of total plant protein extract) per line for homozygous tobacco plants with their standard deviation and standard error of means (SEM).**

| Line / Promoter | data set 1 | | | data set 2 | | |
|---|---|---|---|---|---|---|
| line | average | standard deviation | SEM | average | standard deviation | SEM |
| AA6-03 | 12469 | 8648 | 3867 | 18877 | 12264 | 5485 |
| AA6-05 | 8645 | 4711 | 2355 | 11223 | 6394 | 3197 |
| AA6-06 | 3954 | 826 | 477 | 6169 | 2580 | 1154 |
| AA6-07 | 3070 | 1018 | 509 | 6485 | 3832 | 1916 |
| | | | | | | |
| 35S-06 | 8040 | 4051 | 2025 | 11133 | 6186 | 2765 |
| 35S-09 | 10234 | 2832 | 1416 | 20216 | 7398 | 3699 |
| 35S-13 | 14996 | 4704 | 3326 | 26516 | 17900 | 8005 |
| 35S-25 | 5841 | 2272 | 1016 | 17501 | 9293 | 4156 |
| 35S-28 | 1436 | 10 | 5 | 1404 | 19 | 9 |
| 35S-29 | 9295 | 3996 | 2307 | 25035 | 17459 | 10080 |
| | | | | | | |
| neg control | 1436 | 10 | 5 | 1404 | 19 | 9 |
| average AA6 | 7035 | 5414 | 2707 | 10689 | 7482 | 3741 |
| average 35S | 8307 | 3131 | 1278 | 16968 | 10641 | 4344 |

As can be seen, the average promoter activity of the AA6 promoter (5kb promoter) is essentially equal to that of the 35S promoter in transgenic tobacco leaves (both in plants hemizygous and homozygous for the transgene).

### Expression of GUS in tomato

**Table 3: Average GUS activity (expressed in mU/mg of total plant protein extract) per line for hemizygous tomato plants with their standard deviation and standard error of means (SEM).**

| Line / Promoter | data set 1 | | | data set 2 | | |
|---|---|---|---|---|---|---|
| line | average | standard deviation | SEM | average | standard deviation | SEM |
| AA6-11 | 1126 | 895 | 400 | 2615 | 621 | 278 |
| AA6-29 | 144 | 188 | 77 | 1538 | 739 | 302 |
| AA6-30 | 25302 | 4602 | 2301 | 31004 | 2078 | 929 |
| AA6-31 | 24370 | 4927 | 2463 | 31512 | 2203 | 1102 |
| | | | | | | |
| 35S-01 | 5027 | 8483 | 3794 | 7797 | 9420 | 4213 |
| 35S-04 | 11731 | 2189 | 979 | 11479 | 2157 | 965 |
| 35S-23 | 3024 | 6272 | 2805 | 6988 | 9170 | 4585 |
| 35S-27 | 14310 | 4775 | 2388 | 15625 | 4109 | 1838 |
| | | | | | | |
| neg control | 0 | 0 | 0 | 757 | 875 | 309 |
| overall means AA6 | 12736 | 3129 | 1565 | 16667 | 1520 | 760 |
| overall means 35S | 8523 | 5946 | 2973 | 10472 | 6835 | 3418 |

**Table 4: Average GUS activity (expressed in mU/mg of total plant protein extract) per line for homozygous tomato plants with their standard deviation and standard error of means (SEM).**

| | data set 1 | | | data set 2 | | |
|---|---|---|---|---|---|---|
| line | average | standard deviation | SEM | average | standard deviation | SEM |
| AA6-11 | 1357 | 844 | 377 | 2259 | 574 | 257 |
| AA6-29 | 14 | 28 | 14 | 1279 | 915 | 458 |
| AA6-30 | 35784 | 9382 | 4196 | 39472 | 6631 | 3828 |
| AA6-31 | 36187 | 5942 | 2657 | 41129 | 2247 | 1125 |
| | | | | | | |
| 35S-01 | 10496 | 6385 | 2856 | 11025 | 6425 | 2873 |
| 35S-04 | 13638 | 2642 | 1321 | 13569 | 1219 | 609 |
| 35S-23 | 4551 | 9103 | 4551 | 1659 | 434 | 217 |
| 35S-27 | 19538 | 2080 | 1201 | 20685 | 1128 | 564 |
| | | | | | | |
| neg control | 0 | 0 | 0 | 757 | 875 | 309 |
| overall means AA6 | 18336 | 5713 | 2857 | 21035 | 3134 | 1567 |
| overall means 35S | 12056 | 6001 | 3001 | 11735 | 3583 | 1791 |

As can be seen in Table 4, the average promoter activity of the AA6 promoter (5kb promoter) is significantly higher (in dataset I about 52% higher and in dataset 2 about 79% higher than 35S, when taking 35S activity as standard) in tomato compared to that of the 35S promoter in transgenic tomato leaves (both in plants hemizygous and homozygous for the transgene).

### Example 6 - Promoter activity in other plant species

Comparison of GUS expression driven by AA6 and CaMV35S promoters in other plant species has been tested and confirmed. At present the list of species tested with AA6 promoter driven expression of a transgene includes (besides tobacco and tomato, as shown above) lettuce, melon and *Brassica,* in each of which constitutive expression has been shown.

### Example 7 - re-sequencing of the "3kb" and "5kb" AA6 promoters

The nucleic acid sequences present in vectors pKG8135 and pKG8137 (Fig. 3) were re-sequenced to check the sequences and to resolve ambiguities.
The ambiguous positions are indicated in the sequence listing of SEQ ID NO: 6 ("3kb"promoter) and SEQ ID NO: 7 ("5kb"promoter), wherein also the most likely nucleotide at the ambiguous position is identified (see also the tables below).

Due to ambiguous nucleotides, minimal sequence differences exist: SEQ ID NO: 1 has 99.6% and 99.7% sequence identity to SEQ ID NO: 8 and 6, respectively (using 'needle', Gap opening = 10.0, gap extension =0.5; DNAFull matrix). SEQ ID NO: 2 has 99.6% and 99.8% sequence identity to SEQ ID NO: 9 and 7, respectively (using 'needle', Gap opening = 10.0, gap extension =0.5; DNAFull matrix).

Ambiguities and resolution of ambiguities:

| Nucleotide position in SEQ ID NO: 6 | ambiguity | Most likely nucleotide |
|---|---|---|
| 679 | W (A/T ambiguity) | T |
| 682 | W (A/T ambiguity) | A |
| 683 | W (A/T ambiguity) | T |
| 691 | A inserted | A insertion seem reliable |
| 864 | Y (T/C ambiguity) | T |
| 1269 | R (G/A ambiguity) | A |
| 1559 | R (G/A ambiguity) | A |
| 1560 | M (A/C ambiguity) | c |
| 1561 | D (G/A/T ambiguity) | T |
| 1916 | R (G/A ambiguity) | A |
| 2085 | Y (T/C ambiguity) | T |
| 2101 | Y (T/C ambiguity) | C |
| 2463 | Y (T/C ambiguity) | T |
| 2526 | A should be deleted | A deletion seems reliable |
| 2534 | R (G/A ambiguity) | G |
| 2681 | K (G/T ambiguity) | G |
| 2920 | R (G/A ambiguity) | A |

| Nucleotide position in SEQ ID NO: 7 | ambiguity | Most likely nucleotide |
|---|---|---|
| 35 | G insertion | G insertion seems reliable |
| 240 | Y (T/C ambiguity) | T |
| 768 | W (A/T ambiguity) | A |
| 769 | Y (T/C ambiguity) | T |
| 1558 | R (G/A ambiguity) | A |
| 1742 | Y (T/C ambiguity) | T |
| 2016 | T insertion | T insertion seems reliable |
| 2695 | W (A/T ambiguity) | T |
| 2698 | W (A/T ambiguity) | A |
| 2699 | W (A/T ambiguity) | T |
| 2707 | A inserted | A insertion seem reliable |
| 2880 | Y (T/C ambiguity) | T |
| 3285 | R (G/A ambiguity) | A |
| 3575 | R (G/A ambiguity) | A |
| 3576 | M (A/C ambiguity) | C |
| 3577 | D (G/A/T ambiguity) | T |
| 3932 | R (G/A ambiguity) | A |
| 4101 | Y (T/C ambiguity) | T |
| 4117 | Y (T/C ambiguity) | C |
| 4479 | Y (T/C ambiguity) | T |
| 4542 | A should be deleted | A deletion seems reliable |
| 4550 | R (G/A ambiguity) | G |
| 4697 | K (G/T ambiguity) | G |
| 4936 | R (G/A ambiguity) | A |

## Claims

1. A transgenic plant or plant cell or plant tissue or organ comprising a chimeric gene integrated in its genome, **characterized in that** said chimeric gene comprises a constitutive promoter operably linked to a homologous or heterologous nucleic acid sequence, wherein a homologous nucleic acid sequence is found naturally in said plant or plant cell or plant tissue or organ, and wherein a heterologous nucleic acid sequence is not naturally found in said plant or plant cell or plant tissue or organ, wherein the constitutive promoter is selected from the group of:
(a) the nucleic acid sequence of SEQ ID NO: 1 or SEQ ID NO: 2;
(b) a functional fragment of the nucleic acid sequence of SEQ ID NO: 1 or SEQ ID NO: 2, said functional fragment being capable of conferring constitutive transcription in plant cells, organs and plants;
(c) a nucleic acid sequence comprising at least 70% sequence identity with the nucleic acid sequence of SEQ ID NO: 1 or 2 over the full length, said nucleic acid sequence being capable of conferring constitutive transcription in plant cells, organs and plants;
(d) a functional fragment of the nucleic acid sequence of (c), said functional fragment being capable of conferring constitutive transcription in plant cells, organs and plants,
wherein said promoter is not the promoter of the OASA1 gene of Arabidopsis thaliana, or a fragment thereof.

2. The plant, plant cell, tissue or organ according to claim 1, wherein the promoter activity is reduced by less than 10% when the plant, cell, tissue or organ is exposed to one or more biotic and/or abiotic stresses.

3. An isolated nucleic acid sequence having constitutive promoter activity when introduced into plant cells, wherein said nucleic acid sequence comprising a sequence selected from:
(a) the nucleic acid sequence of SEQ ID NO: 1 or SEQ ID NO: 2;
(b) a fragment of the nucleic acid sequence of SEQ ID NO: 1 or SEQ ID NO: 2;
(c) a nucleic acid sequence comprising at least 70% sequence identity with the nucleic acid sequence of SEQ ID NO: 1 or 2 over the full length, said nucleic acid sequence being capable of conferring constitutive transcription in plant cells, organs and plants;
(d) a fragment of the nucleic acid sequence of (c).

4. A chimeric gene comprising a nucleic acid sequence according to claim 3, operably linked to a sense, antisense, or inverted repeat nucleic acid sequence, and optionally a 3' UTR sequence.

5. The chimeric gene according to claim 4, wherein said 3'UTR sequence is the nucleic acid sequence of SEQ ID NO: 3, or a fragment of the nucleic acid sequence of SEQ ID NO: 3, or a nucleic acid sequence comprising at least 70% nucleic acid identity to the nucleic acid sequence of SEQ ID NO: 3.

6. A vector comprising a nucleic acid sequence according to claim 3, or a chimeric gene according to claim 4 or 5.

7. A transgenic plant or plant cell comprising the chimeric gene according to claim 4 or 5.

8. Use of a promoter selected from the group of:
(a) the nucleic acid sequence of SEQ ID NO: 1 or SEQ ID NO: 2;
(b) a functional fragment of the nucleic acid sequence of SEQ ID NO: 1 or SEQ ID NO: 2, said functional fragment being capable of conferring constitutive transcription in plant cells, organs and plants;
(c) a nucleic acid sequence comprising at least 70% sequence identity with the nucleic acid sequence of SEQ ID NO: 1 or 2 over the full length, said nucleic acid sequence being capable of conferring constitutive transcription in plant cells, organs and plants;
(d) a functional fragment of the nucleic acid sequence of (c), said functional fragment being capable of conferring constitutive transcription in plant cells, organs and plants,
wherein said promoter is not the promoter of the OASA1 gene of Arabidopsis thaliana, or a fragment thereof, for the constitutive expression of a sense and/or antisense nucleic acid sequence in a transgenic plant or plant cell or plant tissue or organ.

9. A method for making a transgenic plant or plant cell, comprising the steps of:
(a) generating a chimeric gene according to claim 4 or 5 or a vector according to claim 6;
(b) transforming a plant or plant cell with said chimeric gene or vector; and, optionally,
(c) regenerating transgenic plants.

10. An isolated nucleic acid molecule comprising the nucleotide sequence of SEQ ID NO: 3.

## Patentansprüche

1. Transgene Pflanze oder Pflanzenzelle oder transgenes Pflanzengewebe oder Organ, umfassend ein in deren bzw. dessen Genom integriertes chimäres Gen, **dadurch gekennzeichnet, dass** das chimäre Gen einen konstitutiven Promotor umfasst, funktionell verknüpft mit einer homologen oder heterologen Nucleinsäuresequenz, wobei eine homologe Nucleinsäuresequenz in der Pflanze oder Pflanzenzelle oder dem Pflanzengewebe oder Organ natürlich vorkommt und wobei eine heterologe Nucleinsäuresequenz in der Pflanze oder Pflanzenzelle oder dem Pflanzengewebe oder Organ nicht natürlich vorkommt, wobei der konstitutive Promotor ausgewählt ist aus der Gruppe:
(a) der Nucleinsäuresequenz der SEQ ID NO: 1 oder SEQ ID NO: 2;
(b) einem funktionellen Fragment der Nucleinsäuresequenz der SEQ ID NO: 1 oder SEQ ID NO: 2, wobei das funktionelle Fragment fähig ist, konstitutive Transkription in Pflanzenzellen, Organen und Pflanzen zu verleihen;
(c) einer Nucleinsäuresequenz, umfassend mindestens 70% Sequenzidentität mit der Nucleinsäuresequenz der SEQ ID NO: 1 oder SEQ ID NO: 2 über die gesamte Länge hinweg, wobei die Nucleinsäuresequenz fähig ist, konstitutive Transkription in Pflanzenzellen, Organen und Pflanzen zu verleihen;
(d) einem funktionellen Fragment der Nucleinsäuresequenz von (c), wobei das funktionelle Fragment fähig ist, konstitutive Transkription in Pflanzenzellen, Organen und Pflanzen zu verleihen,
wobei es sich bei dem Promotor nicht um den Promotor des Gens OASA1 von Arabidopsis thaliana oder ein Fragment davon handelt.

2. Pflanze, Pflanzenzelle, Gewebe oder Organ gemäß Anspruch 1, wobei die Promotoraktivität um weniger als 10% reduziert wird, wenn die Pflanze, die Zelle, das Gewebe oder das Organ einem oder mehreren biotischen und/oder abiotischen Stressen ausgesetzt wird.

3. Isolierte Nucleinsäuresequenz, die konstitutive Promotoraktivität aufweist, wenn sie in Pflanzenzellen eingeführt wird, wobei die Nucleinsäuresequenz eine Sequenz umfasst, ausgewählt aus:
(a) der Nucleinsäuresequenz der SEQ ID NO: 1 oder SEQ ID NO: 2;
(b) einem Fragment der Nucleinsäuresequenz der SEQ ID NO: 1 oder SEQ ID NO: 2;
(c) einer Nucleinsäuresequenz, umfassend mindestens 70% Sequenzidentität mit der Nucleinsäuresequenz der SEQ ID NO: 1 oder SEQ ID NO: 2 über die gesamte Länge hinweg, wobei die Nucleinsäuresequenz fähig ist, konstitutive Transkription in Pflanzenzellen, Organen und Pflanzen zu verleihen;
(d) einem Fragment der Nucleinsäuresequenz von (c).

4. Chimäres Gen, umfassend eine Nucleinsäuresequenz gemäß Anspruch 3, funktionell verbunden mit einer Sense-, Antisense-, oder invertierten Repeat-Nucleinsäuresequenz und gegebenenfalls einer 3'-UTR-Sequenz.

5. Chimäres Gen gemäß Anspruch 4, wobei es sich bei der 3'-UTR-Sequenz um die Nucleinsäuresequenz der SEQ ID NO: 3 oder ein Fragment der Nucleinsäuresequenz der SEQ ID NO: 3 oder eine Nucleinsäuresequenz, die mindestens 70% Nucleinsäureidentität mit der Nucleinsäuresequenz der SEQ ID NO: 3 umfasst, handelt.

6. Vektor, umfassend eine Nucleinsäuresequenz gemäß Anspruch 3 oder ein chimäres Gen gemäß Anspruch 4 oder 5.

7. Transgene Pflanze oder Pflanzenzelle, umfassend das chimäre Gen gemäß Anspruch 4 oder 5.

8. Verwendung eines Promotors, der ausgewählt ist aus der Gruppe:
(a) der Nucleinsäuresequenz der SEQ ID NO: 1 oder SEQ ID NO: 2;
(b) einem funktionellen Fragment der Nucleinsäuresequenz der SEQ ID NO: 1 oder SEQ ID NO: 2, wobei das funktionelle Fragment fähig ist, konstitutive Transkription in Pflanzenzellen, Organen und Pflanzen zu verleihen;
(c) einer Nucleinsäuresequenz, umfassend mindestens 70% Sequenzidentität mit der Nucleinsäuresequenz der SEQ ID NO: 1 oder SEQ ID NO: 2 über die gesamte Länge hinweg, wobei die Nucleinsäuresequenz fähig ist, konstitutive Transkription in Pflanzenzellen, Organen und Pflanzen zu verleihen;
(d) einem funktionellen Fragment der Nucleinsäuresequenz von (c), wobei das funktionelle Fragment fähig ist, konstitutive Transkription in Pflanzenzellen, Organen und Pflanzen zu verleihen,
wobei es sich bei dem Promotor nicht um den Promotor des Gens OASA1 von Arabidopsis thaliana oder ein Fragment davon handelt, zur konstitutiven Expression einer Sense- und/oder Antisense-Nucleinsäuresequenz in einer transgenen Pflanze oder Pflanzenzelle oder einem Pflanzengewebe oder Organ.

9. Verfahren zum Herstellen einer transgenen Pflanze oder Pflanzenzelle, umfassend die Schritte:
(a) Erzeugen eines chimären Gens gemäß Anspruch 4 oder 5 oder eines Vektors gemäß Anspruch 6;
(b) Transformieren einer Pflanze oder Pflanzenzelle mit dem chimären Gen oder Vektor und gegebenenfalls
(c) Regenerieren transgener Pflanzen.

10. Isoliertes Nucleinsäuremolekül, umfassend die Nucleotidsequenz der SEQ ID NO: 3.

## Revendications

1. Plante ou cellule végétale ou tissu ou organe végétal transgénique comprenant un gène chimérique intégré dans son génome, **caractérisé en ce que** ledit gène chimérique comprend un promoteur constitutif lié de manière fonctionnelle à une séquence d'acide nucléique homologue ou hétérologue, dans lequel une séquence d'acide nucléique homologue est naturellement présente dans ladite plante ou cellule végétale ou tissu ou organe végétal, et dans lequel une séquence d'acide nucléique hétérologue n'est pas naturellement présente dans ladite plante ou cellule végétale ou tissu ou organe végétal, dans lequel le promoteur constitutif est choisi dans le groupe de:
(a) la séquence d'acide nucléique de SEQ ID NO: 1 ou SEQ ID NO: 2;
(b) un fragment fonctionnel de la séquence d'acide nucléique de SEQ ID NO: 1 ou SEQ ID NO: 2, ledit fragment fonctionnel étant capable de conférer une transcription constitutive dans les cellules végétales, les organes végétaux et les plantes;
(c) une séquence d'acide nucléique comprenant au moins 70 % d'identité de séquence avec la séquence d'acide nucléique de SEQ ID NO: 1 ou 2 sur toute la longueur, ladite séquence d'acide nucléique étant capable de conférer une transcription constitutive dans les cellules végétales, les organes végétaux et les plantes;
(d) un fragment fonctionnel de la séquence d'acide nucléique de (c), ledit fragment fonctionnel étant capable de conférer une transcription constitutive dans les cellules végétales, les organes végétaux et les plantes,
dans lequel ledit promoteur n'est pas le promoteur du gène OASA1 d'Arabidopsis thaliana, ou un fragment de celui-ci.

2. Plante, cellule végétale, tissu ou organe végétal selon la revendication 1, dans lequel l'activité du promoteur est réduite de moins de 10 % lorsque la plante, la cellule, le tissu ou l'organe végétale est exposé à un ou plusieurs stress biotiques et/ou abiotiques.

3. Séquence d'acide nucléique isolée ayant une activité de promoteur constitutif lorsqu'elle est introduite dans des cellules végétales, dans laquelle ladite séquence d'acide nucléique comprenant une séquence choisie parmi:
(a) la séquence d'acide nucléique de SEQ ID NO: 1 ou SEQ ID NO: 2;
(b) un fragment de la séquence d'acide nucléique de SEQ ID NO: 1 ou SEQ ID NO: 2;
(c) une séquence d'acide nucléique comprenant au moins 70 % d'identité de séquence avec la séquence d'acide nucléique de SEQ ID NO: 1 ou 2 sur toute la longueur, ladite séquence d'acide nucléique étant capable de conférer une transcription constitutive dans les cellules végétales, les organes végétaux et les plantes;
(d) un fragment de la séquence d'acide nucléique de (c).

4. Gène chimérique comprenant une séquence d'acide nucléique selon la revendication 3, liée de manière fonctionnelle à une séquence d'acide nucléique sens, antisens, ou à répétitions inversées, et optionnellement une séquence UTR en 3'.

5. Gène chimérique selon la revendication 4, dans lequel ladite séquence UTR en 3' est la séquence d'acide nucléique de SEQ ID NO: 3, ou un fragment de la séquence d'acide nucléique de SEQ ID NO: 3, ou une séquence d'acide nucléique comprenant au moins 70 % d'identité d'acide nucléique avec la séquence d'acide nucléique de SEQ ID NO: 3.

6. Vecteur comprenant une séquence d'acide nucléique selon la revendication 3, ou un gène chimérique selon la revendication 4 ou 5.

7. Plante ou cellule végétale transgénique comprenant le gène chimérique selon la revendication 4 ou 5.

8. Utilisation d'un promoteur choisi dans le groupe de:
(a) la séquence d'acide nucléique de SEQ ID NO: 1 ou SEQ ID NO: 2;
(b) un fragment fonctionnel de la séquence d'acide nucléique de SEQ ID NO: 1 ou SEQ ID NO: 2, ledit fragment fonctionnel étant capable de conférer une transcription constitutive dans les cellules végétales, les organes végétaux et les plantes;
(c) une séquence d'acide nucléique comprenant au moins 70 % d'identité de séquence avec la séquence d'acide nucléique de SEQ ID NO: 1 ou 2 sur toute la longueur, ladite séquence d'acide nucléique étant capable de conférer une transcription constitutive dans les cellules végétales, les organes végétaux et les plantes;
(d) un fragment fonctionnel de la séquence d'acide nucléique de (c), ledit fragment fonctionnel étant capable de conférer une transcription constitutive dans les cellules végétales, les organes végétaux et les plantes,
dans laquelle ledit promoteur n'est pas le promoteur du gène OASA1 d'Arabidopsis thaliana, ou un fragment de celui-ci, pour l'expression constitutive d'une séquence d'acide nucléique sens et/ou antisens dans une plante ou une cellule végétale ou un tissu ou un organe végétal transgénique.

9. Procédé de fabrication d'une plante ou d'une cellule végétale transgénique, comprenant les étapes de:
(a) génération d'un gène chimérique selon la revendication 4 ou 5 ou d'un vecteur selon la revendication 6;
(b) transformation d'une plante ou d'une cellule végétale avec ledit gène chimérique ou vecteur; et, optionnellement,
(c) régénération des plantes transgéniques.

10. Molécule d'acide nucléique isolée comprenant la séquence nucléotidique de SEQ ID NO: 3.


### SEQUENCE LISTING

<110> KeyGene NV
<120> Constitutive plant promoter
<130> P6005193PCT1
<150> PCT/NL2005/050083
   <151> 2005-12-16
<160> 9
<170> PatentIn version 3.3
<210> 1
   <211> 2986
   <212> DNA
   <213> *Lycopersicon* esculentum
<400> 1
   tggttttcta attctctcat ctcttttctc tgttcttgta tcttatccct ttctgattat     60
   cttcttttgt gttaatctct ccttagttaa tatattatta ctttttgcat gtattccaat    120
   agtaacttat attcaacaat aattatatac aatagtgttt gtattagaat cgagtttatt    180
   tcataaagtc tcgaaagtaa tacattactt tttttagaat aaaaaaaaaa cttgggaata    240
   tggaagttca atctacgcaa aaacctattt tatgatgtca aatgaattca atgtttttac    300
   tcatattgca atataaaaag aacagggcta gtgatttttt ttccttcaaa tgattgctag    360
   ttaattaatg gacttgttct ttcttttgtt tcatattctt gcctttttta agaccttcca    420
   aatcatcaac acaaaaacac cttagacatt attgcatccg acttaattta tatgacataa    480
   aatataaagt ttttttttta aaataaaaat aaagagtttt aaattttata agaaagaaat    540
   atgtatattt aagttcatac taattataaa aacatgattt attaaaaata aatataaaaa    600
   atatatcata taaattaaaa cgaatgaaat atagtatatt aggatgagaa ttcaaaggac    660
   tagaatttcc aaagaaatwt gwwgtttttt atgttttatt agttgacttg ttctatgccg    720
   gtcttgctcg ttcaccatat atagaccaac acttaaagta aatagtggta atttgatttg    780
   acctttcaaa accttcaagg gccaattaga cagaaacttt atattataaa agtaataatg    840
   aatagtagta ataaactaat tattaccaag aaatgatgaa taattgttta tatatatata    900
   tagtgcaata ataattcata accggccgat cctatatatt tattataaaa tatagaagtt    960
   atccaataat ttcatgtctt ttattaaaaa aattcattca ataatttatg tttttctttt   1020
   tacaaagttt aataatagaa cttatttaat taaaactaat gttactatta tgatgaaata   1080
   gtcgagatct cttcattttt cataagagat tcaggattca agctgattat gtgagtgaaa   1140
   tcctaaaaaa tgaaatttgt aatacgtaaa taatacgaat atcaaatacg aaacaaaaag   1200
   aaaattaatg ttattatgat tttgaatttt aattatggta ttttaactac tgaagttgtc   1260
   tcattgaatg tatattataa ttaagcatat acaattccag aaaatatata ttattatcta   1320
   ggtgttcttt taatataata tttgagtttt atgataaaat ttgattaact tttaaaggaa   1380
   aatcttttta aaagtggtca cattagattg agataaatca gaaaggacac gtcacaatct   1440
   aaatatgatt tttttaactt catctataaa tatctttcaa attatataca atgcttacgt 1500
   gaataaatgc tggcactaat gaattaatat ttgtttttaa aataaaatgt tgaaattgmd 1560
   tttagaagtg aacacatgag tcgaagtgtt agaaatagtt actaataaat aatcaatgta 1620
   tttgatataa aaagtgttat taagttgttg ttttgttaga ataactcaaa cattttcatt 1680
   ttatttaatt aaaaattata aatttaaact atatgtataa acaaaatgag caataataaa 1740
   tatagaatga agcaaacatt atgaatttta ttttgaaaaa aaatgatgtg aattataaaa 1800
   atattatata atatttgatc aaacaaattg tttactataa gctaaagaaa tcttgatttt 1860
   aacttataaa tactttactt atcaaatacg taaaaaaatt aaaaattacc tataaatcaa 1920
   tttaatctaa cactttagtc aaacacatta taaatatgaa cttgttgtgc cacgattata 1980
   cacatatatt tttttttatt tattgatata tcctcaaaaa taatgtatct cttaaaatat 2040
   tagtatttat taagatacgg gttcatgaaa tattctttta acttcaaatt tttatttgac 2100
   tttgaaaaca gacccctaaa aagtttaaaa ataacaaaaa ggcaacaaaa aagtttgaaa 2160
   caagaactaa aaaagttaga tgcccaaatc tgcacgataa ggtaagtcaa acaacgacaa 2220
   aatttagtaa attattgatt gtagaaaatg aagcaaatga aggtggctat ccttatataa 2280
   agctatattc agtactctcc tcaacacagt cacagtagat ttcatttctt tgtctttgtc 2340
   aaatcctagg taatttttct ctgcccctta atattttttt ttgctcttga ttgcctttta 2400
   tggaaaattg gttctctgtt catttgacaa gattgctgtt ttgttttttc ttcagtctga 2460
   ttagattttt gctctagatt cgtgggtttt acttgtaaag gtattaggga gtgagtgaga 2520
   tgggaaaatt tcratttttt tttaaaaaaa aattatggga taaaaagtga ttccgagaaa 2580
   tggggttttg ttttttgaga ttgtatgaac tggggatgat ggattcttgg tggtgattga 2640
   ataaaaatgg gatctttaat tattagggat aagattaaag atgctcatgt tcttctttga 2700
   atttgtttgt atgcaaagtt ttgagctttt gggtgttttc cccctaattt agctggcaaa 2760
   tagttgtaat tggtggaagt tggaaaagga aaagaattaa gaagaaattg atgttcattt 2820
   cagtttcttt ttatattttg taaggaacgg aatcaccggt gtcgagtgga atagatttag 2880
   agggttctca aatagttaaa tgagtagctg gtttgatgaa atttgttttg attgtataag 2940
   atttcactta gttttgctat aaatcatcgc agaatcaacc tgatac 2986
<210> 2
   <211> 5000
   <212> DNA
   <213> *Lycopersicon* esculentum
<400> 2
   catataaccc tgaaaattat tttaccattc tttgatcttc tttttcattt tatttgacca     60
   atttataagt attattttaa aagtttaatt ttttttcaaa tttcacgatg aattatggcc    120
   aaatttctcc tagttgtgtt ttgaccataa ataaaaatag aagttgttct ttactttttt    180
   gtgagtaatt tgaagtgatt tttttcctaa gtttttgaaa ttcaacttta atttttttyt    240
   gtttttttgt gacaaggaaa tctgtcttgc tatcttttgg gtgcatacac ggtaaaattc    300
   ctgctcatat gcaataactc actctaactt taagttgaac ttaacaattt tacgattaaa 360
   gtgttttcta aactcaactt catgaaaaaa ttaaaagtta ttgaggaaat tttaaaaata 420
   actaacactg aataatataa ttaaactcct tagctaatgt ttctctaatt acgattcata 480
   gctactatta gaaggagaga agtgagcgac aatatgagag gaggagaaat acgagcgaga 540
   tctgagagag atacaatcaa tttgtgtatc tgattgatac ttgtattatt gttgtaattc 600
   ataataaatt gtatttaatt atatttatct attgccttgt atattgtatt tatgtgttac 660
   catgtatatt tgtgctacca ttttgtattt gtatcattga ctgctatcgt tgtacgtgta 720
   ttgtagaaag agagagagga gaggaatagt gagaagattg ctataawycc aaattataat 780
   tacgaatagt aattctttta aattataatt atgtatagtt aagacatatt ttgtacgttt 840
   atctacattt gccccaaaat tattctcctg tggactctca aagcaggaac caaatagtga 900
   tggattgggc tcctagttct gaacgaattt tgttattggg cttaaagtta aggttggttg 960
   gtcaggtttt tgggtttgca tatctatggg gtagtaaggt ttattctttt ctcgagattc 1020
   gatatttata tttgagcttt attaaatttg gattcacacc aaaaagtttt atattgaggg 1080
   gggggggggg taaagtgctt cctaataaag gcaaaaacga ttccctactt agaagattca 1140
   aactcaaaaa actcgggatg aagtatttac aattctatca cgttgttgat tagttagctt 1200
   tatcattgta tgaggtatac ttgtgggata gaatgttaag acaatttatg aatttctttt 1260
   ttgaatctag aatgtggttt gatgaacaat ggacttaaaa gaagttaatg gaaaatgaga 1320
   aatcaaattt caataaaaat gaagttttta gtgattcttt tcatctatcg ataggtaaaa 1380
   gatatatcat ttggtctaat tcaatcccaa aagttagttt aaagggaaaa gcgggttata 1440
   aagagtacat tcgtctcatt aaccaccaat gtgaactttt attattcttc aacacccatc 1500
   ttattcctag tttttttagc atttggtgtg tgaacaattt tttatttcgg aggtccraca 1560
   tcgggtgaga agagttctac tctgatatca tgtgaaatta ggttttgagc taaacttaca 1620
   ccaaaaagct agcttaaaag atgaaggatt gtacaagcct tataaggaat tcacccgtct 1680
   tattaagcat caatataaaa cttttgtcat tcttaaacaa tataacttcg ataaaaccat 1740
   ycaaataaat atagtataca catgtacaaa gatgtatcta ctcaatgaaa tatatgagcg 1800
   caaatgagat actatgacta ccatcatcgt aaataaaaaa acacataaag tatatattta 1860
   aaggttggtg tttataaagt gtcatgaacc aaactgatcc cgtaaggaca tatgggctat 1920
   tagagagttt tattagggat gcaagtctat tcactcttcg tgaaaaagga ctaggagtta 1980
   gaagagtgat tatgcaaatt attatgtcga agcttggttt tctaattctc tcatctcttt 2040
   tctctgttct tgtatcttat ccctttctga ttatcttctt ttgtgttaat ctctccttag 2100
   ttaatatatt attacttttt gcatgtattc caatagtaac ttatattcaa caataattat 2160
   atacaatagt gtttgtatta gaatcgagtt tatttcataa agtctcgaaa gtaatacatt 2220
   acttttttta gaataaaaaa aaaacttggg aatatggaag ttcaatctac gcaaaaacct 2280
   attttatgat gtcaaatgaa ttcaatgttt ttactcatat tgcaatataa aaagaacagg 2340
   gctagtgatt ttttttcctt caaatgattg ctagttaatt aatggacttg ttctttcttt 2400
   tgtttcatat tcttgccttt tttaagacct tccaaatcat caacacaaaa acaccttaga 2460
   cattattgca tccgacttaa tttatatgac ataaaatata aagttttttt tttaaaataa 2520
   aaataaagag ttttaaattt tataagaaag aaatatgtat atttaagttc atactaatta 2580
   taaaaacatg atttattaaa aataaatata aaaaatatat catataaatt aaaacgaatg 2640
   aaatatagta tattaggatg agaattcaaa ggactagaat ttccaaagaa atwtgwwgtt 2700
   ttttatgttt tattagttga cttgttctat gccggtcttg ctcgttcacc atatatagac 2760
   caacacttaa agtaaatagt ggtaatttga tttgaccttt caaaaccttc aagggccaat 2820
   tagacagaaa ctttatatta taaaagtaat aatgaatagt agtaataaac taattattac 2880
   caagaaatga tgaataattg tttatatata tatatagtgc aataataatt cataaccggc 2940
   cgatcctata tatttattat aaaatataga agttatccaa taatttcatg tcttttatta 3000
   aaaaaattca ttcaataatt tatgtttttt cttttacaaa gtttaataat agaacttatt 3060
   taattaaaac taatgttact attatgatga aatagtcgag atctcttcat ttttcataag 3120
   agattcagga ttcaagctga ttatgtgagt gaaatcctaa aaaatgaaat ttgtaatacg 3180
   taaataatac gaatatcaaa tacgaaacaa aaagaaaatt aatgttatta tgattttgaa 3240
   ttttaattat ggtattttaa ctactgaagt tgtctcattg aatgtatatt ataattaagc 3300
   atatacaatt ccagaaaata tatattatta tctaggtgtt cttttaatat aatatttgag 3360
   ttttatgata aaatttgatt aacttttaaa ggaaaatctt tttaaaagtg gtcacattag 3420
   attgagataa atcagaaagg acacgtcaca atctaaatat gattttttta acttcatcta 3480
   taaatatctt tcaaattata tacaatgctt acgtgaataa atgctggcac taatgaatta 3540
   atatttgttt ttaaaataaa atgttgaaat tgmdtttaga agtgaacaca tgagtcgaag 3600
   tgttagaaat agttactaat aaataatcaa tgtatttgat ataaaaagtg ttattaagtt 3660
   gttgttttgt tagaataact caaacatttt cattttattt aattaaaaat tataaattta 3720
   aactatatgt ataaacaaaa tgagcaataa taaatataga atgaagcaaa cattatgaat 3780
   tttattttga aaaaaaatga tgtgaattat aaaaatatta tataatattt gatcaaacaa 3840
   attgtttact ataagctaaa gaaatcttga ttttaactta taaatacttt acttatcaaa 3900
   tacgtaaaaa aattaaaaat tacctataaa tcaatttaat ctaacacttt agtcaaacac 3960
   attataaata tgaacttgtt gtgccacgat tatacacata tatttttttt tatttattga 4020
   tatatcctca aaaataatgt atctcttaaa atattagtat ttattaagat acgggttcat 4080
   gaaatattct tttaacttca aatttttatt tgactttgaa aacagacccc taaaaagttt 4140
   aaaaataaca aaaaggcaac aaaaaagttt gaaacaagaa ctaaaaaagt tagatgccca 4200
   aatctgcacg ataaggtaag tcaaacaacg acaaaattta gtaaattatt gattgtagaa 4260
   aatgaagcaa atgaaggtgg ctatccttat ataaagctat attcagtact ctcctcaaca 4320
   cagtcacagt agatttcatt tctttgtctt tgtcaaatcc taggtaattt ttctctgccc 4380
   cttaatattt ttttttgctc ttgattgcct tttatggaaa attggttctc tgttcatttg 4440
   acaagattgc tgttttgttt tttcttcagt ctgattagat ttttgctcta gattcgtggg 4500
   ttttacttgt aaaggtatta gggagtgagt gagatgggaa aatttcratt tttttttaaa 4560
   aaaaaattat gggataaaaa gtgattccga gaaatggggt tttgtttttt gagattgtat 4620
   gaactgggga tgatggattc ttggtggtga ttgaataaaa atgggatctt taattattag 4680
   ggataagatt aaagatgctc atgttcttct ttgaatttgt ttgtatgcaa agttttgagc 4740
   ttttgggtgt tttcccccta atttagctgg caaatagttg taattggtgg aagttggaaa 4800
   aggaaaagaa ttaagaagaa attgatgttc atttcagttt ctttttatat tttgtaagga 4860
   acggaatcac cggtgtcgag tggaatagat ttagagggtt ctcaaatagt taaatgagta 4920
   gctggtttga tgaaatttgt tttgattgta taagatttca cttagttttg ctataaatca 4980
   tcgcagaatc aacctgatac 5000
<210> 3
   <211> 347
   <212> DNA
   <213> *Lycopersicon* esculentum
<400> 3
   ctttgtcctt cgattactac atcatcgtcg cgaatagggc ttctgatgaa gcagaatgtt 60
   cttgtctaaa ccttcccctt tccattttgc tgaaactgct agaaaataag gccatctctt 120
   tcctttattg gctatgttct ttaaagtaaa aaaaaaaacc gttcaagtat cttttagtgc 180
   ttttgtaatt ctactgtttc tacaacattt gaaatttata gagctgttgc ttcttgttca 240
   tgaaggtttt tgcttcttgt tccataattt tacgctacgt tgctcggact cttccaaaat 300
   gtccactaat gcgtgttgga ttcttcaaaa atagtgtaac tttgaag 347
<210> 4
   <211> 975
   <212> DNA
   <213> *Lycopersicon* esculentum
<400> 4
   atggcggggg aaaagactgg aattgccaag gatgtaactg aattgatagg taacactcct      60
   ttggtatacc tgaataatgt tgtggatggg tgtgttgcac gtgttgctgc caagctggaa     120
   agcatggagc catgctctag tgttaaggat aggattggtt atagtatgat tacagatgct     180
   gaggagaagg gcttaatcaa acctggcgag agtgtcctca tcgaacctac aagtggaaac     240
   accggtgtag gattggcatt catggctgct gctaaaggct acaaactcat cattacgatg     300
   ccttcttcaa tgagtcttga gagaagaatt attctgcgtg ctttcggtgc tgagttggtg     360
   cttactgacc cagcaaaagg gatgaaaggt gcaatttcga aggccgaaga gataaggggc     420
   aaaacaccta actcctatat tcttcagcaa tttgaaaacc ctgctaaccc aaagatacac     480
   tatgaaacca ctggtcctga gatctggaaa ggctcaaacg ggaaagtgga tgctctagtc     540
   tctggaattg gaacaggagg cacaataaca ggttcaggca agtatttgag agagcagaac     600
   cccaacatca agctgtatgg cgtggaacca gttgaaagtg ctatcctttc tggtggaaag     660
   cctggtccac ataagattca ggggattggt gctggtttcg ttcctggtgt tttggaagtt     720
   aaccttattg acgatgtagt tcaggtttca agtgatgaat ccatagaaat ggctaagctt     780
   ctggcattga aggaaggatt gctagtggga atatcatctg gtgctgctgc tgctgcggca     840
   attaaagttg ctaagcgtcc tgaaaatgct gggaagctca ttgttgttgt tttcccaagc     900
   ttcggggagc gatatctttc ctctgtgctc ttcgaaactg tcagacgaga agcagagaac     960
   atgactgtgg agcct                                                      975
<210> 5
   <211> 325
   <212> PRT
   <213> Lycopersicon esculentum
<400> 5
   Met Ala Gly Glu Lys Thr Gly Ile Ala Lys Asp Val Thr Glu Leu Ile
   1               5                   10                  15
   Gly Asn Thr Pro Leu Val Tyr Leu Asn Asn Val Val Asp Gly Cys Val
               20                  25                  30
   Ala Arg Val Ala Ala Lys Leu Glu Ser Met Glu Pro Cys Ser Ser Val
           35                  40                  45
   Lys Asp Arg Ile Gly Tyr Ser Met Ile Thr Asp Ala Glu Glu Lys Gly
       50                  55                  60
   Leu Ile Lys Pro Gly Glu Ser Val Leu Ile Glu Pro Thr Ser Gly Asn
   65                  70                  75                  80
   Thr Gly Val Gly Leu Ala Phe Met Ala Ala Ala Lys Gly Tyr Lys Leu
                   85                  90                  95
   Ile Ile Thr Met Pro Ser Ser Met Ser Leu Glu Arg Arg Ile Ile Leu
               100                 105                 110
   Arg Ala Phe Gly Ala Glu Leu Val Leu Thr Asp Pro Ala Lys Gly Met
           115                 120                 125
   Lys Gly Ala Ile Ser Lys Ala Glu Glu Ile Arg Gly Lys Thr Pro Asn
       130                 135                 140
   Ser Tyr Ile Leu Gln Gln Phe Glu Asn Pro Ala Asn Pro Lys Ile His
   145                 150                 155                 160
   Tyr Glu Thr Thr Gly Pro Glu Ile Trp Lys Gly Ser Asn Gly Lys Val
                   165                 170                 175
   Asp Ala Leu Val Ser Gly Ile Gly Thr Gly Gly Thr Ile Thr Gly Ser
               180                 185                 190
   Gly Lys Tyr Leu Arg Glu Gln Asn Pro Asn Ile Lys Leu Tyr Gly Val
           195                 200                 205
   Glu Pro Val Glu Ser Ala Ile Leu Ser Gly Gly Lys Pro Gly Pro His
       210                 215                 220
   Lys Ile Gln Gly Ile Gly Ala Gly Phe Val Pro Gly Val Leu Glu Val
   225                 230                 235                 240
   Asn Leu Ile Asp Asp Val Val Gln Val Ser Ser Asp Glu Ser Ile Glu
                   245                 250                 255
   Met Ala Lys Leu Leu Ala Leu Lys Glu Gly Leu Leu Val Gly Ile Ser
               260                 265                 270
   Ser Gly Ala Ala Ala Ala Ala Ala Ile Lys Val Ala Lys Arg Pro Glu
           275                 280                 285
   Asn Ala Gly Lys Leu Ile Val Val Val Phe Pro Ser Phe Gly Glu Arg
       290                 295                 300
   Tyr Leu Ser Ser Val Leu Phe Glu Thr Val Arg Arg Glu Ala Glu Asn
   305                 310                 315                 320
   Met Thr Val Glu Pro
                   325
<210> 6
   <211> 2987
   <212> DNA
   <213> *Lycopersicon* esculentum
<220>
   <221> misc_difference
   <222> (679)..(679)
   <223> w is likely T
<220>
   <221> misc_difference
   <222> (682)..(682)
<223> w is likely A
<220>
   <221> misc_difference
   <222> (683)..(683)
   <223> w is likely T
<220>
   <221> misc_difference
   <222> (691)..(691)
   <223> insertion of A appears reliable
<220>
   <221> misc_difference
   <222> (864)..(864)
   <223> Y is probably T
<220>
   <221> misc_difference
   <222> (1269)..(1269)
   <223> R is probably A
<220>
   <221> misc_difference
   <222> (1559)..(1559)
   <223> R is probably A
<220>
   <221> misc_difference
   <222> (1560)..(1560)
   <223> M is probably C
<220>
   <221> misc_difference
   <222> (1561)..(1561)
   <223> D is probably T
<220>
   <221> misc_difference
   <222> (1916)..(1916)
   <223> R is porbably A
<220>
   <221> misc_difference
   <222> (2085)..(2085)
   <223> Y is probably T
<220>
   <221> misc_difference
   <222> (2101)..(2101)
   <223> Y is probably C
<220>
   <221> misc_difference
   <222> (2463)..(2463)
   <223> Y is probably T
<220>
   <221> misc_difference
   <222> (2526)..(2526)
   <223> A is probably deleted
<220>
   <221> misc_difference
   <222> (2534)..(2534)
   <223> R is probably G
<220>
   <221> misc_difference
   <222> (2681)..(2681)
   <223> K is probably G
<220>
   <221> misc_difference
   <222> (2920)..(2920)
   <223> R is probably A
<400> 6
   tggttttcta attctctcat ctcttttctc tgttcttgta tcttatccct ttctgattat      60
   cttcttttgt gttaatctct ccttagttaa tatattatta ctttttgcat gtattccaat     120
   agtaacttat attcaacaat aattatatac aatagtgttt gtattagaat cgagtttatt     180
   tcataaagtc tcgaaagtaa tacattactt tttttagaat aaaaaaaaaa cttgggaata     240
   tggaagttca atctacgcaa aaacctattt tatgatgtca aatgaattca atgtttttac     300
   tcatattgca atataaaaag aacagggcta gtgatttttt ttccttcaaa tgattgctag     360
   ttaattaatg gacttgttct ttcttttgtt tcatattctt gcctttttta agaccttcca     420
   aatcatcaac acaaaaacac cttagacatt attgcatccg acttaattta tatgacataa     480
   aatataaagt ttttttttta aaataaaaat aaagagtttt aaattttata agaaagaaat     540
   atgtatattt aagttcatac taattataaa aacatgattt attaaaaata aatataaaaa     600
   atatatcata taaattaaaa cgaatgaaat atagtatatt aggatgagaa ttcaaaggac     660
   tagaatttcc aaagaaatwt gwwgtttttt aatgttttat tagttgactt gttctatgcc     720
   ggtcttgctc gttcaccata tatagaccaa cacttaaagt aaatagtggt aatttgattt     780
   gacctttcaa aaccttcaag ggccaattag acagaaactt tatattataa aagtaataat     840
   gaatagtagt aataaactaa ttaytaccaa gaaatgatga ataattgttt atatatatat     900
   atagtgcaat aataattcat aaccggccga tcctatatat ttattataaa atatagaagt     960
   tatccaataa tttcatgtct tttattaaaa aaattcattc aataatttat gttttttctt    1020
   ttacaaagtt taataataga acttatttaa ttaaaactaa tgttactatt atgatgaaat    1080
   agtcgagatc tcttcatttt tcataagaga ttcaggattc aagctgatta tgtgagtgaa    1140
   atcctaaaaa atgaaatttg taatacgtaa ataatacgaa tatcaaatac gaaacaaaaa    1200
   gaaaattaat gttattatga ttttgaattt taattatggt attttaacta ctgaagttgt    1260
   ctcattgart gtatattata attaagcata tacaattcca gaaaatatat attattatct    1320
   aggtgttctt ttaatataat atttgagttt tatgataaaa tttgattaac ttttaaagga    1380
   aaatcttttt aaaagtggtc acattagatt gagataaatc agaaaggaca cgtcacaatc    1440
   taaatatgat ttttttaact tcatctataa atatctttca aattatatac aatgcttacg    1500
   tgaataaatg ctggcactaa tgaattaata tttgttttta aaataaaatg ttgaaattrm    1560
   dtttagaagt gaacacatga gtcgaagtgt tagaaatagt tactaataaa taatcaatgt    1620
   atttgatata aaaagtgtta ttaagttgtt gttttgttag aataactcaa acattttcat    1680
   tttatttaat taaaaattat aaatttaaac tatatgtata aacaaaatga gcaataataa    1740
   atatagaatg aagcaaacat tatgaatttt attttgaaaa aaaatgatgt gaattataaa    1800
   aatattatat aatatttgat caaacaaatt gtttactata agctaaagaa atcttgattt    1860
   taacttataa atactttact tatcaaatac gtaaaaaaat taaaaattac ctataratca    1920
   atttaatcta acactttagt caaacacatt ataaatatga acttgttgtg ccacgattat    1980
   acacatatat ttttttttat ttattgatat atcctcaaaa ataatgtatc tcttaaaata    2040
   ttagtattta ttaagatacg ggttcatgaa atattctttt aactycaaat ttttatttga    2100
   ytttgaaaac agacccctaa aaagtttaaa aataacaaaa aggcaacaaa aaagtttgaa    2160
   acaagaacta aaaaagttag atgcccaaat ctgcacgata aggtaagtca aacaacgaca    2220
   aaatttagta aattattgat tgtagaaaat gaagcaaatg aaggtggcta tccttatata    2280
   aagctatatt cagtactctc ctcaacacag tcacagtaga tttcatttct ttgtctttgt    2340
   caaatcctag gtaatttttc tctgcccctt aatatttttt tttgctcttg attgcctttt    2400
   atggaaaatt ggttctctgt tcatttgaca agattgctgt tttgtttttt cttcagtctg    2460
   atyagatttt tgctctagat tcgtgggttt tacttgtaaa ggtattaggg agtgagtgag   2520
   atgggaaaat ttcrattttt ttttaaaaaa aaattatggg ataaaaagtg attccgagaa   2580
   atggggtttt gttttttgag attgtatgaa ctggggatga tggattcttg gtggtgattg    2640
   aataaaaatg ggatctttaa ttattaggga taagattaaa katgctcatg ttcttctttg   2700
   aatttgtttg tatgcaaagt tttgagcttt tgggtgtttt ccccctaatt tagctggcaa   2760
   atagttgtaa ttggtggaag ttggaaaagg aaaagaatta agaagaaatt gatgttcatt   2820
   tcagtttctt tttatatttt gtaaggaacg gaatcaccgg tgtcgagtgg aatagattta   2880
   gagggttctc aaatagttaa atgagtagct ggtttgatgr aatttgtttt gattgtataa   2940
   gatttcactt agttttgcta taaatcatcg cagaatcaac ctgatac                  2987
<210> 7
   <211> 5003
   <212> DNA
   <213> Lycopersicon esculentum
<220>
   <221> misc_difference
   <222> (35)..(35)
   <223> insertion of G seems reliable
<220>
   <221> misc_difference
   <222> (240)..(240)
   <223> Y is probably T
<220>
   <221> misc_difference
   <222> (768)..(769)
   <223> WY is probably AT
<220>
   <221> misc_difference
   <222> (1558)..(1558)
   <223> R is probably A
<220>
   <221> misc_difference
   <222> (1742)..(1742)
   <223> Y is probably T
<220>
   <221> misc_difference
   <222> (2016)..(2016)
   <223> insertion of T seems reliable
<220>
   <221> misc_difference
   <222> (2695)..(2699)
   <223> WTGWW is probably TTGAT
<220>
   <221> misc_difference
   <222> (2707)..(2707)
   <223> insertion of A seems reliable
<220>
   <221> misc_difference
   <222> (2880)..(2880)
   <223> Y is probably T
<220>
   <221> misc_difference
   <222> (3285)..(3285)
   <223> R is probably A
<220>
   <221> misc_difference
   <222> (3575)..(3577)
   <223> RMD is probably ACT
<220>
   <221> misc_difference
   <222> (3932)..(3932)
   <223> R is probably A
<220>
   <221> misc_difference
   <222> (4101)..(4101)
   <223> Y is probably T
<220>
   <221> misc_difference
   <222> (4117)..(4117)
   <223> Y is probably C
<220>
   <221> mist_difference
   <222> (4479)..(4479)
   <223> Y is probably T
<220>
   <221> misc_difference
   <222> (4542)..(4542)
   <223> A is probably deleted
<220>
   <221> misc_difference
   <222> (4550)..(4550)
   <223> R is probably G
<220>
   <221> misc_difference
   <222> (4697)..(4697)
   <223> K is probably G
<220>
   <221> misc_difference
   <222> (4936)..(4936)
   <223> R is probably A
<400> 7
   catataaccc tgaaaattat tttaccattc tttggatctt ctttttcatt ttatttgacc     60
   aatttataag tattatttta aaagtttaat tttttttcaa atttcacgat gaattatggc    120
   caaatttctc ctagttgtgt tttgaccata aataaaaata gaagttgttc tttacttttt    180
   tgtgagtaat ttgaagtgat ttttttccta agtttttgaa attcaacttt aattttttty    240
   tgtttttttg tgacaaggaa atctgtcttg ctatcttttg ggtgcataca cggtaaaatt    300
   cctgctcata tgcaataact cactctaact ttaagttgaa cttaacaatt ttacgattaa    360
   agtgttttct aaactcaact tcatgaaaaa attaaaagtt attgaggaaa ttttaaaaat    420
   aactaacact gaataatata attaaactcc ttagctaatg tttctctaat tacgattcat    480
   agctactatt agaaggagag aagtgagcga caatatgaga ggaggagaaa tacgagcgag 540
   atctgagaga gatacaatca atttgtgtat ctgattgata cttgtattat tgttgtaatt 600
   cataataaat tgtatttaat tatatttatc tattgccttg tatattgtat ttatgtgtta 660
   ccatgtatat ttgtgctacc attttgtatt tgtatcattg actgctatcg ttgtacgtgt 720
   attgtagaaa gagagagagg agaggaatag tgagaagatt gctataawyc caaattataa 780
   ttacgaatag taattctttt aaattataat tatgtatagt taagacatat tttgtacgtt 840
   tatctacatt tgccccaaaa ttattctcct gtggactctc aaagcaggaa ccaaatagtg 900
   atggattggg ctcctagttc tgaacgaatt ttgttattgg gcttaaagtt aaggttggtt 960
   ggtcaggttt ttgggtttgc atatctatgg ggtagtaagg tttattcttt tctcgagatt 1020
   cgatatttat atttgagctt tattaaattt ggattcacac caaaaagttt tatattgagg 1080
   gggggggggg gtaaagtgct tcctaataaa ggcaaaaacg attccctact tagaagattc 1140
   aaactcaaaa aactcgggat gaagtattta caattctatc acgttgttga ttagttagct 1200
   ttatcattgt atgaggtata cttgtgggat agaatgttaa gacaatttat gaatttcttt 1260
   tttgaatcta gaatgtggtt tgatgaacaa tggacttaaa agaagttaat ggaaaatgag 1320
   aaatcaaatt tcaataaaaa tgaagttttt agtgattctt ttcatctatc gataggtaaa 1380
   agatatatca tttggtctaa ttcaatccca aaagttagtt taaagggaaa agcgggttat 1440
   aaagagtaca ttcgtctcat taaccaccaa tgtgaacttt tattattctt caacacccat 1500
   cttattccta gtttttttag catttggtgt gtgaacaatt ttttatttcg gaggtccrac 1560
   atcgggtgag aagagttcta ctctgatatc atgtgaaatt aggttttgag ctaaacttac 1620
   accaaaaagc tagcttaaaa gatgaaggat tgtacaagcc ttataaggaa ttcacccgtc 1680
   ttattaagca tcaatataaa acttttgtca ttcttaaaca atataacttc gataaaacca 1740
   tycaaataaa tatagtatac acatgtacaa agatgtatct actcaatgaa atatatgagc 1800
   gcaaatgaga tactatgact accatcatcg taaataaaaa aacacataaa gtatatattt 1860
   aaaggttggt gtttataaag tgtcatgaac caaactgatc ccgtaaggac atatgggcta 1920
   ttagagagtt ttattaggga tgcaagtcta ttcactcttc gtgaaaaagg actaggagtt 1980
   agaagagtga ttatgcaaat tattatgtcg aagctttggt tttctaattc tctcatctct 2040
   tttctctgtt cttgtatctt atccctttct gattatcttc ttttgtgtta atctctcctt 2100
   agttaatata ttattacttt ttgcatgtat tccaatagta acttatattc aacaataatt 2160
   atatacaata gtgtttgtat tagaatcgag tttatttcat aaagtctcga aagtaataca 2220
   ttactttttt tagaataaaa aaaaaacttg ggaatatgga agttcaatct acgcaaaaac 2280
   ctattttatg atgtcaaatg aattcaatgt ttttactcat attgcaatat aaaaagaaca 2340
   gggctagtga ttttttttcc ttcaaatgat tgctagttaa ttaatggact tgttctttct 2400
   tttgtttcat attcttgcct tttttaagac cttccaaatc atcaacacaa aaacacctta 2460
   gacattattg catccgactt aatttatatg acataaaata taaagttttt tttttaaaat 2520
   aaaaataaag agttttaaat tttataagaa agaaatatgt atatttaagt tcatactaat 2580
   tataaaaaca tgatttatta aaaataaata taaaaaatat atcatataaa ttaaaacgaa 2640
   tgaaatatag tatattagga tgagaattca aaggactaga atttccaaag aaatwtgwwg 2700
   ttttttaatg ttttattagt tgacttgttc tatgccggtc ttgctcgttc accatatata 2760
   gaccaacact taaagtaaat agtggtaatt tgatttgacc tttcaaaacc ttcaagggcc 2820
   aattagacag aaactttata ttataaaagt aataatgaat agtagtaata aactaattay 2880
   taccaagaaa tgatgaataa ttgtttatat atatatatag tgcaataata attcataacc 2940
   ggccgatcct atatatttat tataaaatat agaagttatc caataatttc atgtctttta 3000
   ttaaaaaaat tcattcaata atttatgttt tttcttttac aaagtttaat aatagaactt 3060
   atttaattaa aactaatgtt actattatga tgaaatagtc gagatctctt catttttcat 3120
   aagagattca ggattcaagc tgattatgtg agtgaaatcc taaaaaatga aatttgtaat 3180
   acgtaaataa tacgaatatc aaatacgaaa caaaaagaaa attaatgtta ttatgatttt 3240
   gaattttaat tatggtattt taactactga agttgtctca ttgartgtat attataatta 3300
   agcatataca attccagaaa atatatatta ttatctaggt gttcttttaa tataatattt 3360
   gagttttatg ataaaatttg attaactttt aaaggaaaat ctttttaaaa gtggtcacat 3420
   tagattgaga taaatcagaa aggacacgtc acaatctaaa tatgattttt ttaacttcat 3480
   ctataaatat ctttcaaatt atatacaatg cttacgtgaa taaatgctgg cactaatgaa 3540
   ttaatatttg tttttaaaat aaaatgttga aattrmdttt agaagtgaac acatgagtcg 3600
   aagtgttaga aatagttact aataaataat caatgtattt gatataaaaa gtgttattaa 3660
   gttgttgttt tgttagaata actcaaacat tttcatttta tttaattaaa aattataaat 3720
   ttaaactata tgtataaaca aaatgagcaa taataaatat agaatgaagc aaacattatg 3780
   aattttattt tgaaaaaaaa tgatgtgaat tataaaaata ttatataata tttgatcaaa 3840
   caaattgttt actataagct aaagaaatct tgattttaac ttataaatac tttacttatc 3900
   aaatacgtaa aaaaattaaa aattacctat aratcaattt aatctaacac tttagtcaaa 3960
   cacattataa atatgaactt gttgtgccac gattatacac atatattttt ttttatttat 4020
   tgatatatcc tcaaaaataa tgtatctctt aaaatattag tatttattaa gatacgggtt 4080
   catgaaatat tcttttaact ycaaattttt atttgayttt gaaaacagac ccctaaaaag 4140
   tttaaaaata acaaaaaggc aacaaaaaag tttgaaacaa gaactaaaaa agttagatgc 4200
   ccaaatctgc acgataaggt aagtcaaaca acgacaaaat ttagtaaatt attgattgta 4260
   gaaaatgaag caaatgaagg tggctatcct tatataaagc tatattcagt actctcctca 4320
   acacagtcac agtagatttc atttctttgt ctttgtcaaa tcctaggtaa tttttctctg 4380
   ccccttaata tttttttttg ctcttgattg ccttttatgg aaaattggtt ctctgttcat 4440
   ttgacaagat tgctgttttg ttttttcttc agtctgatya gatttttgct ctagattcgt 4500
   gggttttact tgtaaaggta ttagggagtg agtgagatgg gaaaatttcr attttttttt 4560
   aaaaaaaaat tatgggataa aaagtgattc cgagaaatgg ggttttgttt tttgagattg 4620
   tatgaactgg ggatgatgga ttcttggtgg tgattgaata aaaatgggat ctttaattat 4680
   tagggataag attaaakatg ctcatgttct tctttgaatt tgtttgtatg caaagttttg 4740
   agcttttggg tgttttcccc ctaatttagc tggcaaatag ttgtaattgg tggaagttgg 4800
   aaaaggaaaa gaattaagaa gaaattgatg ttcatttcag tttcttttta tattttgtaa 4860
   ggaacggaat caccggtgtc gagtggaata gatttagagg gttctcaaat agttaaatga 4920
   gtagctggtt tgatgraatt tgttttgatt gtataagatt tcacttagtt ttgctataaa 4980
   tcatcgcaga atcaacctga tac 5003
<210> 8
   <211> 2986
   <212> DNA
   <213> *Lycopersicon* esculentum
<400> 8
   tggttttcta attctctcat ctcttttctc tgttcttgta tcttatccct ttctgattat 60
   cttcttttgt gttaatctct ccttagttaa tatattatta ctttttgcat gtattccaat 120
   agtaacttat attcaacaat aattatatac aatagtgttt gtattagaat cgagtttatt 180
   tcataaagtc tcgaaagtaa tacattactt tttttagaat aaaaaaaaaa cttgggaata 240
   tggaagttca atctacgcaa aaacctattt tatgatgtca aatgaattca atgtttttac 300
   tcatattgca atataaaaag aacagggcta gtgatttttt ttccttcaaa tgattgctag 360
   ttaattaatg gacttgttct ttcttttgtt tcatattctt gcctttttta agaccttcca 420
   aatcatcaac acaaaaacac cttagacatt attgcatccg acttaattta tatgacataa 480
   aatataaagt ttttttttta aaataaaaat aaagagtttt aaattttata agaaagaaat 540
   atgtatattt aagttcatac taattataaa aacatgattt attaaaaata aatataaaaa 600
   atatatcata taaattaaaa cgaatgaaat atagtatatt aggatgagaa ttcaaaggac 660
   tagaatttcc aaagaaattt gatgtttttt aatgttttat tagttgactt gttctatgcc 720
   ggtcttgctc gttcaccata tatagaccaa cacttaaagt aaatagtggt aatttgattt 780
   gacctttcaa aaccttcaag ggccaattag acagaaactt tatattataa aagtaataat 840
   gaatagtagt aataaactaa ttattaccaa gaaatgatga ataattgttt atatatatat 900
   atagtgcaat aataattcat aaccggccga tcctatatat ttattataaa atatagaagt 960
   tatccaataa tttcatgtct tttattaaaa aaattcattc aataatttat gttttttctt 1020
   ttacaaagtt taataataga acttatttaa ttaaaactaa tgttactatt atgatgaaat 1080
   agtcgagatc tcttcatttt tcataagaga ttcaggattc aagctgatta tgtgagtgaa 1140
   atcctaaaaa atgaaatttg taatacgtaa ataatacgaa tatcaaatac gaaacaaaaa 1200
   gaaaattaat gttattatga ttttgaattt taattatggt attttaacta ctgaagttgt 1260
   ctcattgaat gtatattata attaagcata tacaattcca gaaaatatat attattatct 1320
   aggtgttctt ttaatataat atttgagttt tatgataaaa tttgattaac ttttaaagga 1380
   aaatcttttt aaaagtggtc acattagatt gagataaatc agaaaggaca cgtcacaatc 1440
   taaatatgat ttttttaact tcatctataa atatctttca aattatatac aatgcttacg 1500
   tgaataaatg ctggcactaa tgaattaata tttgttttta aaataaaatg ttgaaattac 1560
   ttttagaagt gaacacatga gtcgaagtgt tagaaatagt tactaataaa taatcaatgt 1620
   atttgatata aaaagtgtta ttaagttgtt gttttgttag aataactcaa acattttcat 1680
   tttatttaat taaaaattat aaatttaaac tatatgtata aacaaaatga gcaataataa 1740
   atatagaatg aagcaaacat tatgaatttt attttgaaaa aaaatgatgt gaattataaa 1800
   aatattatat aatatttgat caaacaaatt gtttactata agctaaagaa atcttgattt 1860
   taacttataa atactttact tatcaaatac gtaaaaaaat taaaaattac ctatagatca 1920
   atttaatcta acactttagt caaacacatt ataaatatga acttgttgtg ccacgattat 1980
   acacatatat ttttttttat ttattgatat atcctcaaaa ataatgtatc tcttaaaata 2040
   ttagtattta ttaagatacg ggttcatgaa atattctttt aacttcaaat ttttatttga 2100
   ctttgaaaac agacccctaa aaagtttaaa aataacaaaa aggcaacaaa aaagtttgaa 2160
   acaagaacta aaaaagttag atgcccaaat ctgcacgata aggtaagtca aacaacgaca 2220
   aaatttagta aattattgat tgtagaaaat gaagcaaatg aaggtggcta tccttatata 2280
   aagctatatt cagtactctc ctcaacacag tcacagtaga tttcatttct ttgtctttgt 2340
   caaatcctag gtaatttttc tctgcccctt aatatttttt tttgctcttg attgcctttt 2400
   atggaaaatt ggttctctgt tcatttgaca agattgctgt tttgtttttt cttcagtctg 2460
   atcagatttt tgctctagat tcgtgggttt tacttgtaaa ggtattaggg agtgagtgag 2520
   atgggaaatt tcgatttttt tttaaaaaaa aattatggga taaaaagtga ttccgagaaa 2580
   tggggttttg ttttttgaga ttgtatgaac tggggatgat ggattcttgg tggtgattga 2640
   ataaaaatgg gatctttaat tattagggat aagattaaat atgctcatgt tcttctttga 2700
   atttgtttgt atgcaaagtt ttgagctttt gggtgttttc cccctaattt agctggcaaa 2760
   tagttgtaat tggtggaagt tggaaaagga aaagaattaa gaagaaattg atgttcattt 2820
   cagtttcttt ttatattttg taaggaacgg aatcaccggt gtcgagtgga atagatttag 2880
   agggttctca aatagttaaa tgagtagctg gtttgatgga atttgttttg attgtataag 2940
   atttcactta gttttgctat aaatcatcgc agaatcaacc tgatac 2986
<210> 9
   <211> 5002
   <212> DNA
   <213> Lycopersicon esculentum
<400> 9
   catataaccc tgaaaattat tttaccattc tttggatctt ctttttcatt ttatttgacc     60
   aatttataag tattatttta aaagtttaat tttttttcaa atttcacgat gaattatggc     120
   caaatttctc ctagttgtgt tttgaccata aataaaaata gaagttgttc tttacttttt     180
   tgtgagtaat ttgaagtgat ttttttccta agtttttgaa attcaacttt aatttttttt     240
   tgtttttttg tgacaaggaa atctgtcttg ctatcttttg ggtgcataca cggtaaaatt     300
   cctgctcata tgcaataact cactctaact ttaagttgaa cttaacaatt ttacgattaa 360
   agtgttttct aaactcaact tcatgaaaaa attaaaagtt attgaggaaa ttttaaaaat 420
   aactaacact gaataatata attaaactcc ttagctaatg tttctctaat tacgattcat 480
   agctactatt agaaggagag aagtgagcga caatatgaga ggaggagaaa tacgagcgag 540
   atctgagaga gatacaatca atttgtgtat ctgattgata cttgtattat tgttgtaatt 600
   cataataaat tgtatttaat tatatttatc tattgccttg tatattgtat ttatgtgtta 660
   ccatgtatat ttgtgctacc attttgtatt tgtatcattg actgctatcg ttgtacgtgt 720
   attgtagaaa gagagagagg agaggaatag tgagaagatt gctataaatc caaattataa 780
   ttacgaatag taattctttt aaattataat tatgtatagt taagacatat tttgtacgtt 840
   tatctacatt tgccccaaaa ttattctcct gtggactctc aaagcaggaa ccaaatagtg 900
   atggattggg ctcctagttc tgaacgaatt ttgttattgg gcttaaagtt aaggttggtt 960
   ggtcaggttt ttgggtttgc atatctatgg ggtagtaagg tttattcttt tctcgagatt 1020
   cgatatttat atttgagctt tattaaattt ggattcacac caaaaagttt tatattgagg 1080
   gggggggggg gtaaagtgct tcctaataaa ggcaaaaacg attccctact tagaagattc 1140
   aaactcaaaa aactcgggat gaagtattta caattctatc acgttgttga ttagttagct 1200
   ttatcattgt atgaggtata cttgtgggat agaatgttaa gacaatttat gaatttcttt 1260
   tttgaatcta gaatgtggtt tgatgaacaa tggacttaaa agaagttaat ggaaaatgag 1320
   aaatcaaatt tcaataaaaa tgaagttttt agtgattctt ttcatctatc gataggtaaa 1380
   agatatatca tttggtctaa ttcaatccca aaagttagtt taaagggaaa agcgggttat 1440
   aaagagtaca ttcgtctcat taaccaccaa tgtgaacttt tattattctt caacacccat 1500
   cttattccta gtttttttag catttggtgt gtgaacaatt ttttatttcg gaggtccaac 1560
   atcgggtgag aagagttcta ctctgatatc atgtgaaatt aggttttgag ctaaacttac 1620
   accaaaaagc tagcttaaaa gatgaaggat tgtacaagcc ttataaggaa ttcacccgtc 1680
   ttattaagca tcaatataaa acttttgtca ttcttaaaca atataacttc gataaaacca 1740
   ttcaaataaa tatagtatac acatgtacaa agatgtatct actcaatgaa atatatgagc 1800
   gcaaatgaga tactatgact accatcatcg taaataaaaa aacacataaa gtatatattt 1860
   aaaggttggt gtttataaag tgtcatgaac caaactgatc ccgtaaggac atatgggcta 1920
   ttagagagtt ttattaggga tgcaagtcta ttcactcttc gtgaaaaagg actaggagtt 1980
   agaagagtga ttatgcaaat tattatgtcg aagctttggt tttctaattc tctcatctct 2040
   tttctctgtt cttgtatctt atccctttct gattatcttc ttttgtgtta atctctcctt 2100
   agttaatata ttattacttt ttgcatgtat tccaatagta acttatattc aacaataatt 2160
   atatacaata gtgtttgtat tagaatcgag tttatttcat aaagtctcga aagtaataca 2220
   ttactttttt tagaataaaa aaaaaacttg ggaatatgga agttcaatct acgcaaaaac 2280
   ctattttatg atgtcaaatg aattcaatgt ttttactcat attgcaatat aaaaagaaca 2340
   gggctagtga ttttttttcc ttcaaatgat tgctagttaa ttaatggact tgttctttct 2400
   tttgtttcat attcttgcct tttttaagac cttccaaatc atcaacacaa aaacacctta 2460
   gacattattg catccgactt aatttatatg acataaaata taaagttttt tttttaaaat 2520
   aaaaataaag agttttaaat tttataagaa agaaatatgt atatttaagt tcatactaat 2580
   tataaaaaca tgatttatta aaaataaata taaaaaatat atcatataaa ttaaaacgaa 2640
   tgaaatatag tatattagga tgagaattca aaggactaga atttccaaag aaatttgatg 2700
   ttttttaatg ttttattagt tgacttgttc tatgccggtc ttgctcgttc accatatata 2760
   gaccaacact taaagtaaat agtggtaatt tgatttgacc tttcaaaacc ttcaagggcc 2820
   aattagacag aaactttata ttataaaagt aataatgaat agtagtaata aactaattac 2880
   taccaagaaa tgatgaataa ttgtttatat atatatatag tgcaataata attcataacc 2940
   ggccgatcct atatatttat tataaaatat agaagttatc caataatttc atgtctttta 3000
   ttaaaaaaat tcattcaata atttatgttt tttcttttac aaagtttaat aatagaactt 3060
   atttaattaa aactaatgtt actattatga tgaaatagtc gagatctctt catttttcat 3120
   aagagattca ggattcaagc tgattatgtg agtgaaatcc taaaaaatga aatttgtaat 3180
   acgtaaataa tacgaatatc aaatacgaaa caaaaagaaa attaatgtta ttatgatttt 3240
   gaattttaat tatggtattt taactactga agttgtctca ttgagtgtat attataatta 3300
   agcatataca attccagaaa atatatatta ttatctaggt gttcttttaa tataatattt 3360
   gagttttatg ataaaatttg attaactttt aaaggaaaat ctttttaaaa gtggtcacat 3420
   tagattgaga taaatcagaa aggacacgtc acaatctaaa tatgattttt ttaacttcat 3480
   ctataaatat ctttcaaatt atatacaatg cttacgtgaa taaatgctgg cactaatgaa 3540
   ttaatatttg tttttaaaat aaaatgttga aattactttt agaagtgaac acatgagtcg 3600
   aagtgttaga aatagttact aataaataat caatgtattt gatataaaaa gtgttattaa 3660
   gttgttgttt tgttagaata actcaaacat tttcatttta tttaattaaa aattataaat 3720
   ttaaactata tgtataaaca aaatgagcaa taataaatat agaatgaagc aaacattatg 3780
   aattttattt tgaaaaaaaa tgatgtgaat tataaaaata ttatataata tttgatcaaa 3840
   caaattgttt actataagct aaagaaatct tgattttaac ttataaatac tttacttatc 3900
   aaatacgtaa aaaaattaaa aattacctat aaatcaattt aatctaacac tttagtcaaa 3960
   cacattataa atatgaactt gttgtgccac gattatacac atatattttt ttttatttat 4020
   tgatatatcc tcaaaaataa tgtatctctt aaaatattag tatttattaa gatacgggtt 4080
   catgaaatat tcttttaact ccaaattttt atttgacttt gaaaacagac ccctaaaaag 4140
   tttaaaaata acaaaaaggc aacaaaaaag tttgaaacaa gaactaaaaa agttagatgc 4200
   ccaaatctgc acgataaggt aagtcaaaca acgacaaaat ttagtaaatt attgattgta 4260
   gaaaatgaag caaatgaagg tggctatcct tatataaagc tatattcagt actctcctca 4320
   acacagtcac agtagatttc atttctttgt ctttgtcaaa tcctaggtaa tttttctctg 4380
   ccccttaata tttttttttg ctcttgattg ccttttatgg aaaattggtt ctctgttcat 4440
   ttgacaagat tgctgttttg ttttttcttc agtctgatta gatttttgct ctagattcgt 4500
   gggttttact tgtaaaggta ttagggagtg agtgagatgg gaaatttcga ttttttttta 4560
   aaaaaaaatt atgggataaa aagtgattcc gagaaatggg gttttgtttt ttgagattgt 4620
   atgaactggg gatgatggat tcttggtggt gattgaataa aaatgggatc tttaattatt 4680
   agggataaga ttaaagatgc tcatgttctt ctttgaattt gtttgtatgc aaagttttga 4740
   gcttttgggt gttttccccc taatttagct ggcaaatagt tgtaattggt ggaagttgga 4800
   aaaggaaaag aattaagaag aaattgatgt tcatttcagt ttctttttat attttgtaag 4860
   gaacggaatc accggtgtcg agtggaatag atttagaggg ttctcaaata gttaaatgag 4920
   tagctggttt gatgaaattt gttttgattg tataagattt cacttagttt tgctataaat 4980
   catcgcagaa tcaacctgat ac 5002